# EUROPÄISCHE PATENTSCHRIFT

(11) **EP 2 744 474 B1**
(45) Veröffentlichungstag und Bekanntmachung des Hinweises auf die Patenterteilung: **07.10.2015**
(21) Anmeldenummer: 12746329.7
(22) Anmeldetag: 10.08.2012
(51) Int. Cl.: A61K 8/30, A61K 9/06, A61K 9/107, A61K 31/00, A61K 47/06, A61K 47/12, A61Q 19/00

(54) **MEDIZINISCHE HAUTSCHUTZ-ZUSAMMENSETZUNG MIT EINER DIE HAUTBARRIERE VERBESSERNDEN WIRKSTOFFKOMBINATION**
MEDICINAL SKIN PROTECTION COMPOSITION WITH AN ACTIVE INGREDIENT COMBINATION WHICH IMPROVES THE SKIN BARRIER
COMPOSITION MÉDICALE DE PROTECTION DE LA PEAU COMPRENANT UNE COMBINAISON DE PRINCIPES ACTIFS AMÉLIORANT LA BARRIÈRE CUTANÉE

(30) Priorität: 18.08.2011 DE 102011110909
(43) Veröffentlichungstag der Anmeldung: 25.06.2014
(73) Patentinhaber: Paul Hartmann AG, 89522 Heidenheim (DE); Deb IP Limited, Denby, Derbyshire DE5 8JZ (GB)
(72) Erfinder: SMOLA, Hans, 66121 Saarbrücken (DE); KAGAN-HOPER, Marina, 89073 Ulm (DE); HEISLER, Eckhard, 47608 Geldern (DE); ALLEF, Petra, 45128 Essen (DE); MANGEN, Thomas, 40545 Düsseldorf (DE)
(74) Vertreter: Keller, Günter
(86) Internationale Anmeldenummer: PCT/EP2012/065658
(87) Internationale Veröffentlichungsnummer: WO 2013/024012

(56) Entgegenhaltungen:
- WO-A1-2006/036557
- DE-A1-102007 028 027
- DE-A1-102007 031 452
- GHADIALLY R ET AL: "The aged epidermal permeability barrier. Structural, functional, and lipid biochemical abnormalities in humans and a senescent murine model", JOURNAL OF CLINICAL INVESTIGATION, AMERICAN SOCIETY FOR CLINICAL INVESTIGATION, US, Bd. 95, Nr. 5, 1. Mai 1995 (1995-05-01), Seiten 2281-2290, XP002522060, ISSN: 0021-9738, DOI: 10.1172/JCI117919
- VISSCHER MARTY O ET AL: "Effect of soaking and natural moisturizing factor on stratum corneum water-handling properties", JOURNAL OF COSMETIC SCIENCE, SOCIETY OF COSMETIC CHEMISTS, NEW YORK, NY, US, Bd. 54, Nr. 3, 1. Januar 2003 (2003-01-01) , Seiten 289-300, XP009097810, ISSN: 1525-7886

## Beschreibung

Die vorliegende Erfindung betrifft Zusammensetzungen, die eine Wirkstoffkombination aus Mandelöl, Leinölfettsäuren, Aminosäuren und Kreatin enthalten, für den Einsatz zu medizinisch-dermatologischen Zwecken. Die Zusammensetzungen sind zur Reinigung, Pflege und zum Schutz der menschlichen Haut, insbesondere von barrieregeschädigter Haut geeignet.

Mit zunehmendem Alter weist die Haut ein wachsendes Ungleichgewicht zwischen dem Aufbau und dem Abbau von Collagen, einem der hauptsächlichen Bestandteile der Dermis, in Richtung Abbau auf. Ursache hierfür sind unter anderem eine niedrige Rate an neu synthetisiertem Collagen und eine erhöhte Aktivität von Collagen abbauenden Enzymen, wie Collagenase MMP-1. Außerdem nimmt die Fähigkeit der Haut zur Regeneration mit zunehmendem Alter ab. Daher benötigt die reife Haut, insbesondere, wenn sie ungünstigen Umgebungseinflüssen, wie Druck, mangelnde Belüftung oder Feuchtigkeit ausgesetzt ist, einer speziellen Pflege. Solche ungünstigen Einflüsse treten häufig bei körperlichen Fehlfunktionen, wie Inkontinenz, auf.

Die erfindungsgemäßen Produkte bieten Menschen mit barrieregeschädigter Haut vor allem eine schützende und pflegende Wirkung. Das Bedürfnis für derartige Produkte besteht insbesondere bei sogenannter Altershaut.

Unter Altershaut wird die sichtbare Alterung der Haut ab dem 50. Lebensjahr, insbesondere ab dem 65. Lebensjahr verstanden. Sie umfasst sowohl die natürliche, das heißt genetisch programmierte, sogenannte intrinsische Alterung oder Zeitalterung, als auch die durch physikalische, chemische und mikrobiologische Noxen bedingte, sogenannte extrinsische Alterung oder Lichtalterung. Die Hautalterung geht mit einer strukturellen Reduktion der Haut einher, das heißt, die Haut wird in allen Schichten dünner und ärmer an Zellen und funktionstragenden Strukturen. Damit geht die Barrierefunktion der Haut zum Teil verloren, siehe Hautkrankheiten und Hautpflege im Alter, O.P. Hornstein, Wissenschaftliche Verlagsgesellschaft mbH, Stuttgart, 2002.

Symptome der Altershaut sind trockene Haut, hartnäckiger Juckreiz, bedingt durch die trockene Haut (Norman RA. Geriatric dermatology. Dermatol Ther. 2003;16:260-8) oder auch unabhängig davon, Falten und Pigmentflecken. Besonders die Trockenheit der Haut (Xerosis) kann zu intensivem Pruritus führen (Norman RA. Xerosis and pruritus in the elderly: recognition and management. Dermatol Ther. 2003;16:254-9) und als sensitive Haut im Einzelfall wahrgenommen werden. Neuere Arbeiten legen nahe, dass trockene Haut mit Mutationen im Filaggin-Gen vergesellschaftet sind (Sergeant et al., Heterozygous null alleles in filaggrin contribute to clinical dry skin in young adults and the elderly. J Invest Dermatol. 2009 129:1042-5). Filaggrin Mutationen sind wiederum in Zusammenhang mit dem Natural Moisturizing Factor, NMF, gebracht worden (Kezic et al., Natural moisturizing factor components in the stratum corneum as biomarkers of filaggrin genotype: evaluation of minimally invasive methods. Br J Dermatol. 2009 161:1098-104) und können gut trockene Haut und die damit einhergehende Barrierestörung erklären. NMF wird durch den Abbau von Filaggrin zu Aminosäuren, die unter anderem zu 2-pyrrolidone-5-carboxylic acid (PCA) und urocanic acid (UCA) im Stratum corneum weiter abgebaut werden. Betrachtet man das Aminogramm von Profilaggrin (NCBI Reference Sequence: NP_002007.1), aus dem durch Proteolyse Filaggrin in den obersten Hautschichten entsteht, fallen spezielle Unterschiede gegenüber humanem Serumalbumin (NCBI Reference Sequence: NP_000468.1) bei der relativen Verteilung der darin enthaltenen Aminosäuren auf. NMF, aber auch Aminosäuren, werden durch Waschprozeduren oder Okklusion reduziert und werden in der Erholungsphase neu gebildet (Robinson et al., Natural moisturizing factors (NMF) in the stratum corneum (SC). I. Effects of lipid extraction and soaking. J Cosmet Sci. 2010 61:13-22. Robinson et al., Natural moisturizing factors (NMF) in the stratum corneum (SC). II. Regeneration of NMF over time after soaking. J Cosmet Sci. 2010 61:23-9. Visscher et al., Regional variation in the free amino acids in the stratum corneum. Int J Cosmet Sci. 2010 61:303-9. Visscher et al., Stratum corneum free amino acids following barrier perturbation and repair. Int J Cosmet Sci. 2011 33:80-9.).

Die DE 10 2007 031 452 offenbart feuchtigkeitsspendende kosmetische und dermatologische Zusammensetzungen mit einer Kombination verschiedener Enhancer, die zur Behandlung von trockener Haut die Wirkung herkömmlicher Feuchthaltemittel von außen deutlich verbessern. Weiterhin zählt diese Veröffentlichung eine nahezu unübersehbare Menge verschiedener weiterer Zusatzstoffe auf.

Die US 2008/0193393 offenbart ein Verabreichungssystem für topisch aufgetragene Komponenten, wobei dieses System zwingend eine Fettsäure, ein Phospholipid und ein Öl in einem bestimmten Verhältnis von Gewichtsprozenten zueinander enthält.

Die US 2008/0268077 offenbart ein Verfahren zur Stärkung der Barrierefunktion von geschädigter Haut, bei dem eine Mischung eines Ceramids und/oder eines Pseudoceramids zusammen mit einem gegen Hautirritationen wirkenden Wirkstoff, wie Bisabolol, Panthenol, Ingwerextrakten und Mischungen hiervon eingesetzt werden soll.

Die US 2010/0286102 beschreibt kosmetische oder pharmazeutische Formulierungen für Haut oder Haar, die ein oder mehrere Ceramide und/oder Pseudoceramide und (alpha-)Bisabolol enthalten.

Ghadially et al., The Journal of Clinical Investigation, Inc. (1995), S. 2281-2290, beschreibt die Barrierefunktion der menschlichen Haut. Mit zunehmendem Alter wird die menschliche Haut im Vergleich zu der jungen Haut problematischer im Hinblick auf die Wiederherstellung der Barrierefunktion nach Irritationen. Dies wird gezeigt bei Reizung der Haut mit einem Lösungsmittel (Aceton), sowie bei mechanischen Irritationen (Klebstreifentest).

Die Hautpflege im Alter, insbesondere die Hautpflege immobiler und/oder an Harn-und/oder Stuhlinkontinenz und/oder an Dekubitus und anderen Wunderkrankungen leidender Menschen stellt vor diesem Hintergrund eine besondere Herausforderung dar.

Ziel der Hautpflege im Alter ist es, die nachlassende Barrierefunktion der Haut zu regenerieren und zu stärken. Außerdem sollen die begleitenden Symptome wie trockene Haut und Juckreiz oder Hautreizungen vermindert werden. Dies ist vor allem auch dann erforderlich, wenn die Haut bei immobilen und/oder an Harn- und/oder Stuhlinkontinenz und/oder an Dekubitus und anderen Wunderkrankungen leidenden Menschen einerseits nur noch zu einer verminderten physiologischen Regeneration zum Beispiel aufgrund einer verminderten Durchblutung im Stande ist und andererseits die Haut zusätzlich durch das mit dem Tragen von Inkontinenzprodukten verbundene Klima negativen Einflüssen ausgesetzt ist.

Erfindungsgemäß werden medizinische Hautschutz-Zusammensetzungen zur verbesserten Hautpflege im Alter bereitgestellt, die eine Wirkstoffkombination enthaltend Mandelöl, Leinölfettsäuren, Aminosäuren und Kreatin in einem dermatologisch verträglichen Träger beinhalten. Dabei berücksichtigt die Zusammensetzung physiologische Gesichtspunkte. Eine erfindungsgemäße medizinische Hautschutz-Zusammensetzung umfasst:
Eine erfindungsgemäße Wirkstoffkombination für die Pflege der Altershaut bestehend aus folgenden Komponenten:
   a) 0,01 bis 20 Gew.-% Mandelöl,
   b) 0,01 bis 1,5 Gew.-% Leinölfettsäuren,
   c) 0,001 bis 0,5 Gew.-% Aminosäuren und
   d) 0,01 bis 1,5 Gew.-% Kreatin,
wobei sich die angegebenen Gewichtsprozente jeweils auf die Gesamtzusammensetzung des Endproduktes beziehen.

In bevorzugten Ausführungsformen besteht die Wirkstoffkombination aus 0,05 bis 18 Gew.-%, weiter bevorzugt aus 0,1 bis 15 Gew.-% Mandelöl und besonders bevorzugt aus 0,1 bis 6 Gew.-% Mandelöl; 0,02 bis 1,2 Gew.-%, bevorzugt 0,05 bis 0,8 Gew.-% Leinölfettsäuren und besonders bevorzugt aus 0,1 bis 0,7 Gew.-% Leinölfettsäuren; 0,002 bis 0,3 Gew.-%, bevorzugt 0,005 bis 0,15 Gew.-% Aminosäuren und besonders bevorzugt aus 0,05 bis 0,1 Gew.-% Aminosäuren und 0,02 bis 1,2 Gew.-%, bevorzugt 0,05 bis 0,8 Gew.-% Kreatin und besonders bevorzugt aus 0,1 bis 0,75 Gew.-% Kreatin, wobei sich die Gewichtsprozente jeweils auf die Gesamtzusammensetzung des Endproduktes beziehen. Die Komponenten des Endproduktes addieren sich auf 100 Gew.-%.

Die erfindungsgemäße Wirkstoffkombination wird in Produkte eingearbeitet, bei denen es sich um medizinische Hautschutz-Zusammensetzungen zur topischen Anwendung handelt. Derartige Endprodukte enthalten über die erfindungsgemäße Wirkstoffkombination hinaus weitere Inhaltsstoffe, wie sie in Zusammensetzungen zur topischen Anwendung üblich sind. Dazu zählen beispielhaft Ölkomponenten, Emulgatoren, Lösungsvermittler, Konservierungsmittel, Antioxidantien, Viskositäts- und pH-Regulierer, Farb- und Duftstoffe, sowie Wasser. Mit diesen Inhaltsstoffen ergänzt sich die erfindungsgemäße Wirkstoffkombination zu 100 Gew.-% der Gesamtzusammensetzung. Es ist dabei nicht erforderlich, dass die einzelnen Komponenten der erfindungsgemäßen Wirkstoffkombination zuerst zusammengestellt bzw. zusammengemischt werden, sondern die einzelnen Komponenten können, je nach den Erfordernissen des Herstellungsverfahrens, auch nacheinander oder gegebenenfalls als Mischungen von zwei oder drei Komponenten mit den anderen Inhaltsstoffen in die Produkte eingearbeitet werden.

Alternativ beinhaltet die erfindungsgemäße medizinische Hautpflege-Zusammensetzung für die Pflege der Altershaut eine Wirkstoffkombination aus folgenden Komponenten:
a) 0,1 bis 15 Gew.-% Mandelöl,
b) 0,05 bis 0,8 Gew.-% Fettsäuren pflanzlichen Ursprungs mit einem omega6 zu omega3 Fettsäure Verhältnis von 1 zu >1
c) 0,005 bis 0,15 Gew.-% Aminosäuren, ausgewählt aus einer oder mehrerer der Aminosäuren Arginin Arg, Glutamin Gln, Histidin His und Serin Ser und/oder mindestens einen Natural Moisturizing Factor, vorzugsweise Pyrrolidincarbonsäure PCA und/oder Urocaninsäure UCA.
d) 0,05 bis 0,8 Gew.-% Kreatin.

In bevorzugter Ausführungsform beinhaltet eine erfindungsgemäße Wirkstoffkombination Mandelöl, das einen Gehalt von etwa 70-80 % Ölsäure, 10-20 % Linolsäure sowie 5-10 % Palmitinsäure und gegebenenfalls noch untergeordnete Reste anderer Säuren aufweist.

Mandelöl ist ein fettes Öl, das durch Kaltpressung oder Extraktion und anschließendes Raffinieren aus süßen Mandeln des Mandelbaums gewonnen wird (prunus amygdalus dulcis). Hauptsächlich besteht das Mandelöl aus Triestern des Glycerins. Da das Öl einen hohen Anteil an ungesättigten Fettsäuren aufweist, wird es schnell ranzig und ist lichtempfindlich. Um das Ranzigwerden des Produktes zu verhindern, muss das Mandelöl vor Oxidation durch Luftsauerstoff sowie Licht geschützt werden. Bei der Verarbeitung ist es daher vorteilhaft, unter Stickstoffgasatmosphäre zu arbeiten oder es müssen Antioxidantien zugesetzt werden, die hinsichtlich der Hautverträglichkeit geeignet sind.

Weiterer wesentlicher Bestandteil der erfindungsgemäßen Wirkstoffkombination sind Leinölfettsäuren. Leinöl ist ein Pflanzenöl, das aus Leinsamen gewonnen wird. Essentielle freie Fettsäuren pflanzliche Ursprungs mit einem omega6 zu omega3 Verhältnis von 1 zu >1 (z.B. nach http://efaeducation.nih.gov/sig/esstable.html zuletzt aufgerufen am 09.08.2011) sollen die Haut mit essentiellen Fettsäuren versorgen, wobei die Freisetzung aus Triglyceriden aus Gründen der Bioverfügbarkeit umgangen werden soll. Die Aufnahme der freien, essentiellen Fettsäuren kann damit direkt über fatty acid transport protein-1, -2, -3, -4, -5, -6 oder fatty acid translocase/CD36 erfolgen (zusammengefasst in Feingold. Thematic review series: skin lipids. The role of epidermal lipids in cutaneous permeability barrier homeostasis. J Lipid Res. 2007 48:2531-46).

Ziel ist es, die entzündungsfördernde Wirkung von omega6 Fettsäuren durch Auswahl geeigneter Ausgangsstoffe zu reduzieren. Darüber hinaus haben manche freien Fettsäuren einen wachstumshemmenden Effekt auf pathogene Bakterien (Übersicht in Desbois et al., Antibacterial free fatty acids: activities, mechanisms of action and biotechnological potential. Appl Microbiol Biotechnol. 2010 85:1629-42.), die die Physiologie der Haut stören können. Zusammen mit den anti-inflammatorischen Eigenschaften des Fettsäuregemischs durch einen hohen omega3 Anteil wird eine synergistische Aktivität aus Normalisierung der Hautflora und geringer Entzündungsaktivität der Haut erzielt. Für diesen synergistischen Effekt muss die Darreichungsform in Form freier Fettsäuren und nicht als Fettsäure-Triglycerid gewährleistet sein.

Unter Leinölfettsäuren (INCI: linseed acid, CAS: CAS 68424-45-3, EINECS No. 270-307-6) werden freie Fettsäuren verstanden, die aus Leinöl gewonnen werden, welches aus den Samen des Leins kalt oder heiß unter Druck gepresst wird. Je nach Art und Herkunft des Leinsamens und dem Verfahren der Ölgewinnung schwankt die natürliche Zusammensetzung des Leinöls und der daraus gewonnenen Leinölfettsäuren. Das aus Leinsamen gewonnene Fettsäuregemisch enthält gesättigte und ein- und mehrfach ungesättigte Fettsäuren und zeichnet sich durch einen hohen Gehalt an omega3 Fettsäuren aus. Die in der erfindungsgemäßen Wirkstoffkombination eingesetzten Leinölfettsäuren weisen ein Verhältnis von omega6 zu omega3 Fettsäuren von 1 zu >1, vorzugsweise von 1 zu >2 vorzugsweise von 1 zu > 2,5, vorzugsweise von 1 zu größer gleich 3 und vorzugsweise von höchstens 1 zu 5 auf.

Außerdem werden unter Leinölfettsäuren solche Fettsäure-Mischungen verstanden, die anderen natürlichen, insbesondere pflanzlichen Ursprungs sind und ein Verhältnis von omega6 zu omega3 Fettsäuren von 1 zu >1 aufweisen. Damit sind Wirkstoffkombinationen umfasst, die auf pflanzlichen Ölen mit geringerem omega3 Fettsäureanteil basieren, wie beispielsweise Hanföl (omega6 zu omega3: ca. 3:1), Rapsöl (2:1) oder Sojaöl (7:1), und die mit omega3 Fettsäuren angereichert werden, um ein Verhältnis von omega6 zu omega3 Fettsäuren von 1 zu >1 zu erreichen. Omega-3 bedeutet, dass die letzte Doppelbindung in der mehrfach ungesättigten Kohlenstoffkette der Fettsäure bei der - von dem Carboxy-Ende aus gesehen - drittletzten Kohlenstoff-Kohlenstoff-Bindung vorliegt. Entsprechend weisen die Omega-6-Fettsäuren an der sechsten Position - vom Omega-Ende her gesehen - die erste Doppelbindung auf.

Bei den Leinölfettsäuren handelt es sich bevorzugt um ein Gemisch aus den folgenden Fettsäuren (Zahl der C-Atome : Zahl der Doppelbindungen in Klammern) in den jeweils angegebenen relativen Anteilen:

| | |
|---|---|
| Palmitinsäure (C16:0) | 4-8 %, |
| Stearinsäure (C18:0) | 2-5 %, |
| Ölsäure (C18:1) | 16-26%, |
| Linolsäure (C18:2) | 14-24 %, |
| Linolensäure (C18:3) | 43-55 % sowie |

Fettsäuren mit Kohlenstoffketten, die mehr als 18 Kohlenstoffatome aufweisen 0,1-2 %; wobei sich die Prozentangaben zu 100 % addieren.

Der Gehalt an omega3 Fettsäuren in Form von Linolensäure liegt bei 43-55 %, der Gehalt an omega6 Fettsäuren in Form von Linolsäure liegt bei 14-24 % (jeweils bezogen auf die gesamte Leinölfettsäure-Komponente), das Verhältnis omega6 zu omega3 Fettsäuren liegt bei 1:2 bis 1:4.

Eine bevorzugte Leinölfettsäure-Komponente ist erhältlich unter dem Namen "Cremerac Polyungesättigte Fettsäuren Destillierte Leinölfettsäure" von der Firma Cremer Oleo GmbH & Co. KG, Hamburg.

Die freien Fettsäuren weisen eine antimikrobielle Aktivität auf. Sie unterstützen die hauteigene Flora in der Aufrechterhaltung des Säureschutzmantels der Haut. Die Verbesserung der Barrierewirkung des Wirkstoffkomplexes auf die Altershaut wird maßgeblich durch die Leinölfettsäuren verursacht. Dies wird in einer in vitro Rekonstruktion humaner Epidermis nachgewiesen. Die Übertragung dieses im in vitro Modell beobachteten Effekts auf die Anwendung an Altershaut führt dazu, dass sich eine kompakte Hautstruktur ausbildet, die von Vorteil ist, um eine Versorgung der äußeren Hautschichten mit Feuchtigkeit von innen zu ermöglichen.

Wird die in vitro rekonstruierte humane Epidermis einem Detergenz ausgesetzt, so wird ein signifikanter Verlust der Lebensfähigkeit der Zellen ( > 50 % ) nach 240 Minuten beobachtet, die im Standardnährmedium ausdifferenziert sind, wohingegen die Zellen aus einem Leinölfettsäure-haltigen Nährmedium wesentlich robuster gegenüber dem Detergenz sind und keinen signifikanten Rückgang der Lebensfähigkeit zeigen, sogar nicht nach 360 Minuten. Die Lebensfähigkeit der Zellen aus einem Leinölfettsäure-haltigen Nährmedium ist 50 % erhöht gegenüber Zellen aus einem Standardnährmedium.

Eine weitere Komponente der Wirkstoffkombination stellen 0,005 bis 0,15 Gew.-% Aminosäuren dar. Aminosäuren sind für die Haut von größter Bedeutung, da sie zum einen Wasser effektiv speichern können und somit für die Regulierung der Hautfeuchte relevant sind und zum anderen als natürlicher Schutz der Haut gegen freie Radikale dienen.

In bevorzugter Ausführungsform enthält die Aminosäure-Komponente mindestens eine oder mehrere der Aminosäuren Arginin Arg, Glutamin Gln, Histidin His und Serin Ser und/oder mindestens einen Natural Moisturizing Factor, vorzugsweise Pyrrolidincarbonsäure PCA und/oder Urocaninsäure UCA. In einer weiter bevorzugten Ausführungsform enthält die Aminosäure-Komponente mindestens die Aminosäuren Arginin Arg, Glutamin Gln, Histidin His und Serin Ser, vorzugsweise mindestens zu 15 % bezogen auf den Anteil der Aminosäuren.

Eine Supplementierung mit Aminosäuren, die der speziellen Komposition von Profillagrin Rechnung trägt und zumindest die Aminosäuren Arginin Arg, Glutamin Gln, Histidin His, Serin Ser und Pyrrolidincarbonsäure PCA als Vorläufer und Bestandteile des NMF enthält, ist besonders bevorzugt. Ebenso soll vorzugsweise ein Faktor des NMF bereits in aktiver Form vorliegen wie z.B. Pyrrolidincarbonsäure PCA (2-pyrrolidone-5-carboxylic acid und/oder Urocaninsäure UCA (urocanic acid).

Daher ist es besonders vorteilhaft, wenn die erfindungsgemäße Wirkstoffkombination mindestens die Aminosäuren Arginin Arg, Glutamin Gln, Histidin His, Serin Ser und weiter bevorzugt, zusätzlich zu diesen Aminosäuren, Pyrrolidincarbonsäure PCA und / oder UCA (urocanic acid) enthält. Zusammen mit den freien Fettsäuren wird so die Physiologie der Hautoberfläche gestärkt.

Weitere, vornehmlich essentielle Aminosäuren können supplementiert werden, die für den Aufbau bzw. die Regeneration der epidermalen Barriere als topische Ernährung dienen (nutritiver Effekt).

Vorzugsweise ist der Gehalt an Arginin, Histidin und Serin in einer Mischung aus Aminosäuren bei bis zu 20 % (bezogen auf alle Aminosäuren der Mischung, entsprechend 100 %). L-Arginin als metabolischer Harnstoff-Vorläufer wird nach seiner Umwandlung zu Harnstoff ebenfalls im Stratum corneum angereichert und führt damit zu einer Verminderung der Hauttrockenheit, jedoch mit dem Vorteil, dass bei Applikation von L-Arginin auf vorgeschädigter Haut irritative Nebenwirkungen vermieden werden, die bei direkter Applikation von Harnstoff vorkommen können.

Ein bevorzugter Aminosäurekomplex enthält:

| | |
|---|---|
| L-Arginin | 8,0% |
| L-Aspartamsäure | 5,2% |
| L-Pyrrolidondecarboxylsäure | 4,27% |
| Glycin | 1,28% |
| L-Alanin | 1,2% |
| L-Serin | 0,8% |
| L-Valin | 0,64% |
| L-Prolin | 0,4% |
| L-Threonin | 0,4% |
| L-Isoleucin | 0,4% |
| L-Histidin | 0,16% |
| L-Phenylalanin | 0,16% |

Er ist erhältlich als 50%ige wässrige Lösung (weitere Inhaltstoffe 15 % Natrium-L-Pyrrolidoncarboxylat, 12 % Natriumlactat) unter dem Namen "Prodew 500" von der Firma Ajinomoto Co., Inc., Tokio, Japan.

Die vierte Komponente der erfindungsgemäßen Wirkstoffkombination besteht in 0,05 bis 0,8 Gew.-%, bezogen auf die fertige Gesamtzusammensetzung an Kreatin. Kreatin ist eine organische Säure, die im menschlichen Körper in der Niere, der Leber und der Bauchspeicheldrüse synthetisiert wird. Kreatin wirkt mit den anderen Bestandteilen der Wirkstoffkombination zusammen und verbessert die pflegende Aktivität der Produkte.

Kreatin kann der Formulierung zugesetzt werden, besonders, wenn die entzündliche Reaktion von Hautzellen durch oxidativen Stress oder andere Auslöser reduziert werden soll (Lenz et al., The creatine kinase system in human skin: protective effects of creatine against oxidative and UV damage in vitro and in vivo. J Invest Dermatol. 2005 124:443-52). Dieses Konzept ist besonders sinnvoll, wenn die Formulierung sowohl Kreatin als auch freie Fettsäuren mit niedrigem pro-inflammatorischem Potential, wie dies bei einem niedrigen Gehalt an omega6 Fettsäuren gegeben ist, in Kombination enthält. Dies ist besonders bei Hautschutzprodukten gewünscht.

Eine erfindungsgemäße medizinische Hautschutz-Zusammensetzung enthält eine Wirkstoffkombination wie beschrieben. Die jeweils angegebenen Anteile der einzelnen Komponenten der Wirkstoffkombination beziehen sich als Gewichtsprozent auf die Gesamtzusammensetzung. Selbstverständlich addieren sich die einzelnen Komponenten der Wirkstoffkombination zusammen mit den übrigen Bestandteilen der medizinischen Hautschutz-Zusammensetzung immer auf 100 Gew.-%.

In einer weiteren, bevorzugten Ausführungsform beinhaltet die erfindungsgemäße medizinische Hautschutz-Zusammensetzung nur die jeweiligen Komponenten der Wirkstoffkombination in den angegebenen Gewichtsprozenten sowie weitere für die Stabilität und Funktion der Zusammensetzung erforderliche Hilfsstoffe. Falls nur bevorzugte Vertreter der einzelnen Komponenten vorhanden sind, werden bevorzugt keine weiteren, weniger bevorzugten Vertreter der jeweiligen Komponenten hinzugefügt. Bei diesen bevorzugten Ausführungsformen beinhalten die medizinischen Hautschutz-Zusammensetzungen dann nur die bevorzugten Vertreter der jeweiligen Komponenten, nicht aber noch weitere, weniger bevorzugte Vertreter dieser Komponenten. In derartigen bevorzugten Fällen besteht die Wirkstoffkombination nur aus den jeweils bevorzugten Vertretern dieser Komponenten.

Die erfindungsgemäße Wirkstoffkombination wird bevorzugt in eine medizinische Hautschutz-Zusammensetzung eingearbeitet, die als Wasser-in-Öl-Emulsion (W/O) oder als Wasser-in-Öl-in-Wasser-Emulsion (W/O/W) vorliegt. Bei den erfindungsgemäßen Zusammensetzungen handelt es sich in besonders bevorzugter Ausführungsform um Lotionen, Cremes und Schäume, die neben den anderen üblichen Bestandteilen einer topischen Zusammensetzung die erfindungsgemäße Wirkstoffkombination beinhalten.

Bei der Formulierung der medizinischen Hautschutz-Zusammensetzungen ist darauf abzustellen, dass der Hautphysiologie der Altershaut Rechnung getragen wird. Daher haben die Zusammensetzungen einen pH Wert zwischen 4,0 und 6,5, insbesondere zwischen 4,5 und 6,0 und weiter insbesondere zwischen 4,8 bis 5,1.

Ein pH-Wert, der sich an den unterschiedlichen Bedürfnissen der Hautphysiologie orientiert, reicht von pH 4.0 bis pH 6,5. pH 4,0, wenn eine optimierte biochemische Synthese- und Prozessierungsleistung des Stratum corneums gewünscht wird (Hachem et al., Acute acidification of stratum corneum membrane domains using polyhydroxyl acids improves lipid processing and inhibits degradation of corneodesmosomes. J Invest Dermatol. 2010 130:500-10). pH 6,5, wenn z.B. im Inguinalbereich eine adaptierte Formulierung gewünscht wird (in Altmeyer Hrsg, Therapielexikon Dermatologie und Allergologie. S. 695, 2. Aufl. 2005, Springer Verlag Berlin Heidelberg). In diesem Zusammenhang sind Arbeiten von Bedeutung, die zeigen konnten, dass sich der Oberflächen-pH-Wert der Haut im Alter deutlich von junger Haut unterscheidet (Choi et al., Stratum corneum acidification is impaired in moderately aged human and murine skin. J Invest Dermatol. 2007 127:2847-56). Ebenso hat sich gezeigt, dass sich andere Erkrankungen auf den pH-Wert der Haut auswirken können (Yosipovitch et al., Skin surface pH in intertriginous areas in NIDDM patients. Possible correlation to candidal intertrigo. Diabetes Care. 1993 16:560-3). Daraus leitet sich die Notwendigkeit einer adaptierten pH-Wert Einstellung der Produkte ab.

Die vier Bestandteile der Wirkstoffkombination, nämlich Mandelöl (a), Leinölfettsäuren (b), Aminosäuren (c) und Kreatin (d) liegen erfindungsgemäß in bestimmten Verhältnissen zueinander vor. Der Anteil der Wirkstoffkombination an der Gesamtzusammensetzung kann in Abhängigkeit von dem jeweiligen Produkt schwanken. Der jeweilige Anteil der Wirkstoffkombination hängt nicht zuletzt von dem Verwendungszweck des fertigen Produktes ab. Bei stark belasteter Haut kann der Gehalt der Wirkstoffkombination höher sein als bei weniger stark belasteter Haut. Der Anteil der Wirkstoffkombination an der Gesamtzusammensetzung liegt zwischen 0,001 und 25 Gew.-%, vorzugsweise zwischen 0,01 und 20 Gew.-%, weiter bevorzugt zwischen 0,1 und 18 Gew.-% und ganz besonders bevorzugt zwischen 1,0 und 18,0 Gew.-%.

In einer bevorzugten Ausführungsform der vorliegenden Erfindung ist die Wirkstoffkombination in eine W/O-Emulsion in Form einer Körperlotion zu einem Anteil von 1,0 bis 25 Gew.-%, vorzugsweise 2,5 bis 20 Gew.-%, weiter bevorzugt 5,0 bis 18 Gew.-% eingearbeitet und das Verhältnis von Aminosäuren:Leinölfettsäuren:Kreatin:Mandelöl liegt in einem Bereich von 0,5:2:4:100 bis 0,5:4:5:100.

In einer bevorzugten Ausführungsform der vorliegenden Erfindung ist die Wirkstoffkombination in eine W/O-Emulsion in Form einer Hautschutzcreme eingearbeitet und das Verhältnis der Komponenten Aminosäuren:Leinölfettsäuren:Kreatin:Mandelöl liegt zwischen 0,5:4:6:10 und 0,5:4:5:27 und der Anteil dieser Komponenten an der Gesamtzusammensetzung liegt bei 0,5 bis 8,0 Gew.-%, vorzugsweise bei 0,75 bis 7,0 Gew.-%, weiter bevorzugt bei 1,0 bis 6,0 Gew.-%. Die Hautschutzcreme hat vorzugsweise einen Gehalt an Zinkoxid von 15 bis 25 Gew.-%, vorzugsweise 18 bis 23 Gew.-%.

In einer bevorzugten Ausführungsform der vorliegenden Erfindung ist die Wirkstoffkombination in eine transparente Hautschutzcreme, die in Form einer W/O/W-Emulsion vorliegt, eingearbeitet. Die Wirkstoffkombination liegt in einem Anteil von 0,05 bis 2,0 Gew.-%, vorzugsweise 0,1 bis 1,7 Gew.-%, weiter bevorzugt 0,2 bis 1,3 Gew.-% bezogen auf die Gesamtzusammensetzung der transparenten Hautschutzcreme vor, wobei sich das Verhältnis Aminosäuren:Leinölfettsäuren:Kreatin:Mandelöl von 0,5:1:1:2 bis 0,5:1:1:5 bewegt. Die transparente Hautschutzcreme ist frei von Zinkoxid und bildet einen transparenten Schutzfilm auf der Haut.

In einer anderen bevorzugten Ausführungsform betrifft die vorliegende Erfindung einen Schaum, der 0,1 bis 4,0 Gew.-%, vorzugsweise 0,2 bis 3,5 Gew.-%, weiter bevorzugt 0,3 bis 3,0 Gew.-% der Wirkstoffkombination bezogen auf die fertige Gesamtzusammensetzung aufweist. Die einzelnen Komponenten Aminosäuren:Leinölfettsäuren:Kreatin:Mandelöl, bewegen sich in dem Bereich von 0,0125:1:1:1 bis 0,125:1:1:1. Um einen derartigen Schaum zu erzeugen, wird die flüssige Phase in eine Sprühdose verbracht. Bei Gebrauch wird die Flüssigkeit mit Hilfe des Treibmittels aufgeschäumt und der pflegende Schaum wird auf die betroffenen Körperpartien aufgebracht.

Die erfindungsgemäßen medizinischen Hautschutz-Zusammensetzungen mit einer Wirkstoffkombination führen in der topischen Anwendung zu einer Schutzwirkung gegenüber wässrigen Schadstoffen. In der visuellen Beurteilung der getesteten Haut treten deutlich geringere Hautschädigungen als bei unbehandelter Haut auf. Die Barrierefunktion der Haut kann durch die topische Anwendung der erfindungsgemäßen Wirkstoffkombination besser aufrecht erhalten werden. Eine Barriereschädigung der Haut, die zu einer erhöhten Verdunstung von Wasser durch die obersten Hautschichten führt, wird vermindert. Der transepidermale Wasserverlust (TEWL) der Haut liegt bei mit den erfindungsgemäßen Wirkstoffkombinationen behandelter Haut unter dem Referenzwert von 11 g/hm² für wässrigen Schadstoffen ausgesetzter, unbehandelter Haut, vorzugsweise unter 10 g/hm², insbesondere zwischen 3 g/hm² und 9 g/hm².

Gegenstand der Erfindung ist auch die Verwendung einer medizinischen Hautschutz-Zusammensetzung mit folgenden Komponenten:
a) 0,001 bis 40 Gew.-% Mandelöl,
b) 0,001 bis 5,0 Gew.-% Fettsäuren pflanzlichen Ursprungs mit einem omega6 zu omega3 Fettsäure Verhältnis von 1 zu >1
c) 0,001 bis 1,0 Gew.-% Aminosäuren und
d) 0,001 bis 5,0 Gew.-% Kreatin,
zur topischen Anwendung auf Altershaut.

Gegenstand der Erfindung ist weiterhin die Verwendung der erfindungsgemäßen medizinischen Hautschutz-Zusammensetzungen, die diese Wirkstoffkombinationen enthalten, zur topischen Anwendung auf Altershaut.

Die Verwendung erfolgt insbesondere auf durch Bettlägerigkeit und/oder Harn- und/oder Stuhlinkontinenz und/oder Dekubitus und andere Wunderkrankungen beanspruchter Altershaut.

Die topische Anwendung dient der Reinigung, Pflege und dem Schutz der Altershaut, insbesondere von durch das Tragen von Inkontinenzprodukten beanspruchter Haut oder von periläsionaler Haut. Dabei sind die Zusammensetzungen Körperregion-spezifisch konzeptioniert, dass heißt, sie sind auf die Applikation am ganzen Körper und/oder auf die periläsionale Haut und/oder den Intimbereich, insbesondere den durch Inkontinenz beanspruchten Intimbereich abgestimmt. Der Intimbereich umfasst den unteren Bauch- und den unteren Rückenbereich, insbesondere den Genital- und Analbereich und das Perineum.

Die transparente Hautschutzcreme und der Schaum sind indiziert als Barriereschutz im Intimbereich, besonders bei Inkontinenz, nach jeder Intim-Reinigung oder -Pflege. Die Anwendung erfolgt auf gereinigte und intakte Haut durch gleichmäßiges Verteilen der Produkte im Intimbereich. Der Schaum wird dazu vor Gebrauch geschüttelt und bevorzugt auf einen Handschuh oder ein anderes Hilfsmittel zum Auftragen des Schaums gesprüht. Die Dosierung erfolgt in einer erbsen- bis haselnussgroßen Menge.

Die Körperlotion ist indiziert als intensive Pflege nach dem Waschen, Duschen oder Baden, wobei die Anwendung gleichmäßig verteilt auf dem ganzen Körper erfolgt und die Dosierung individuell an die Körpergröße und den Hautzustand angepasst wird.

Die Verwendung erfolgt auf der Altershaut vorzugsweise in Kombination mit der Verwendung von absorbierenden Hygieneprodukten, insbesondere Inkontinenzartikeln, Wundauflagen und Krankenunterlagen.

Die erfindungsgemäßen Hautschutz-Zusammensetzungen haben einen positiven Einfluss auf die Qualität von Inkontinenzprodukten, belegt an Run-off Tests sowie Messungen der Einsickerzeiten und der Rücknässung. Durch die Verwendung der erfindungsgemäßen medizinischen Hautschutz-Zusammensetzungen in Kombination mit Inkontinenzartikeln werden die Rücknässungseigenschaften der Inkontinenzprodukte positiv beeinflusst, so dass die Rücknässung fast vollständig unterbleibt. Dies wirkt sich wiederum positiv auf den Hautzustand der Hautpartien aus, die mit dem Inkontinenzartikel in Berührung kommen.

In einer anderen Ausführungsform kann es sich darüber hinaus als sinnvoll und geeignet erweisen, die Wirkstoffkombination in orale Darreichungsformen, etwa als Dragee oder Tablette als Endprodukt, beispielsweise in Form eines Nahrungsergänzungsmittels zu formulieren. Die Wirkung auf die Haut erfolgt nicht topisch, sondern nach oraler Verabreichung von innen und kann begleitend oder alternativ zu einer äußerlichen Anwendung der medizinischen Hautschutz- Zusammensetzung erfolgen.

In einer weiteren Ausführungsform liegt die medizinische Hautschutz-Zusammensetzung mit einer oben beschriebenen Wirkstoffkombination in Form einer Beschichtung vor, die auf die der Haut des Benutzers zugewandten Lage von absorbierenden Hygieneartikeln aufgebracht ist. Inkontinenzartikel wie Windeln, Windelhosen, Binden etc. haben auf derjenigen Seite, die der Haut des Benutzers zugewandt ist, eine Abdeck(Vlies)-Schicht, die die Trennung zwischen dem absorbierenden Material (beispielsweise verschiedene Polyacrylate) von der Körperoberfläche bewirkt.

Es ist bekannt, insbesondere die der Haut zugewandte Lage von absorbierenden Hygieneartikeln, dass heißt insbesondere die sogenannten Topsheets, mit Avivagen auszurüsten, die sich positiv auf die Hautgesundheit auswirken und gleichzeitig das Absorptionsverhalten des Hygieneartikels nicht negativ beeinflussen.

Bei der Herstellung von derartigen Hygieneprodukten wird die medizinische Hautschutz-Zusammensetzung mit einer Wirkstoffkombination entweder als Mischung oder die einzelnen Komponenten werden nacheinander auf die Vliesschicht, beispielsweise durch Sprühen, aufgebracht. Die medizinische Hautschutz-Zusammensetzung mit einer Wirkstoffkombination wird vorzugsweise mit einer inerten Trägerphase appliziert. Die erfindungsgemäßen Verhältnisse der einzelnen Komponenten zueinander, wie für Lotionen und Cremes angegeben, werden dabei eingehalten und die Menge der Wirkstoffkombination, bezogen auf die Abdeckschicht, bewegt sich in einer Menge von 0,01 bis 20 Gew.-% bezogen auf die beschichtete Abdeckschicht.

Die Leistungskennzahlen des absorbierenden Hygieneartikels werden dadurch nicht negativ beeinflusst. Vielmehr ist es so, dass die Verwendung der Wirkstoffkombination in einer Avivage für einen absorbierenden Hygieneartikel, vorzugsweise einen Inkontinenzartikel, die Rücknässung reduziert. Gleichzeitig wird die Hautbarrierefunktion in den Hautarealen gestärkt, in denen der Träger des absorbierenden Hygieneartikels mit dem mit einer Wirkstoffkombination-haltigen Avivage ausgestatteten absorbierenden Hygieneartikel, vorzugsweise einem Inkontinenzartikel, in Kontakt kommt.

Die Erfindung wird durch die nachfolgenden Beispiele näher erläutert, wobei die Komponenten der Wirkstoffkombination, ebenso wie alle anderen Inhaltsstoffe handelsübliche Ausgangsmaterialien für topische Zusammensetzungen sind.

### Beispiel 1:

### Rezeptur 1: Körperlotion (W/O Emulsion)

| | | | |
|---|---|---|---|
| Aqua (Wasser) | 50-70 | % | Lösungsmittel |
| Isopropylmyristat | 5-15 | % | weichmachendes/rückfettendes/ verteilendes Mittel |
| Capryl/Caprin Triglycerid | 5-15 | % | Ölkomponente/rückfettendes Mittel |
| Prunus Amygdalus Dulcis (Süßmandel) Öl | 5-15 | % | Weichmacher, Feuchtigkeitsspender/ Pflegekomponente |
| Glycerin 85% | 0,5-5 | % | Anfeuchter/Feuchtigkeitsspender |
| Cetyl PEG/PPG-10/1 Dimethicone | 0,5-5 | % | Emulgierungsmittel |
| Kaliumsorbat | 0,1-0,8 | % | Konservierungsmittel |
| Natriumbenzoat | 0,1-0,8 | % | Konservierungsmittel |
| Zitronensäure | 0,4-0,9 | % | pH-Regulierer |
| Natriumhydroxid | 0,2-0,8 | % | pH-Regulierer |
| Kreatin | 0,2-0,8 | % | Feuchtigkeitsspender |
| Natriumchlorid | 0,2-0,8 | % | Viskosität-erhöhendes Mittel |
| hydriertes Castor-Öl | 0,2-0,8 | % | Konsistenz-Faktor |
| Cera Alba (Bienenwachs) | 0,1-0,6 | % | Konsistenz-Faktor/Weichmacher |
| Parfüm (Duftstoff) | 0,1-0,6 | % | Duftstoff |
| Leinölfettsäure | 0,1-0,6 | % | Feuchtigkeitsspender /Ölkomponente |
| Aminosäure-Komplex | 0,05-0,15 | % | Anfeuchter/Protein-Aufbaustoff |
| Tocopherol | 0,01-0,04 | % | Vit. E/ Antioxidationsmittel/ Feuchtigkeitsspender |
| Rosmarinus Officinalis (Rosmarin) Blatt Extrakt | 0,005-0,015 | % | Antioxidationsmittel |

Die Menge der einzelnen Komponenten wird so eingestellt, dass deren Summe 100 Gew.-% ergibt.

Herstellung der Körperlotion:

### a) Fettphase

In der Fryma (Homogenisierer) werden die Rohstoffe: Mandelöl, hydriertes Castor-Öl, Capryl/Caprin Triglycerid, Isopropylmyristat, Cetyl PEG/PPG-10/1 Dimethicone, Wachs gebleicht, Leinölfettsäure destilliert, Rosmarinextrakt und Mischtocopherolkonzentrat vorgelegt und auf 85°C erwärmt. Bei einer Rührwerkeinstellung von 10U/Min. wird die Temperatur etwa 15 Minuten gehalten.

### b) Wasserphase

Der Unimix wird mit ca. 150 kg Wasser sterilisiert, anschließend wird auf 55°C abgekühlt. Ein Teil heißes Wasser wird für die Vorlösungen herausgelassen: Natriumchlorid, Citronensäure werden im heißen Wasser gelöst. Natronlauge 50% wird im heißen Wasser gelöst. Glycerin 85%, Aminosäure Komplex werden im heißen Wasser gelöst. Kaliumsorbat wird im heißen Wasser gelöst.

Die Vorlösungen werden in dem Unimix bei 30U/Min. einziehen gelassen. Das restliche Wasser, gereinigt - bis auf 10% - wird in dem Unimix einziehen gelassen und gut gerührt, bis eine homogene Lösung entstanden ist (ca. 2 Min./1400 U).

Dann wird Kreatin einziehen gelassen und die restlichen 10 % gereinigtes Wasser nachgezogen. Homogenisation in 2 Min. bei 1400 U. Der pH Wert wird kontrolliert (Sollwert 4,8 - 5,1).

### c) Parfümölphase

Das Parfümöl wird abgewogen und bereitgestellt.

### d) Emulsionsbildung

Die kalte Wasserphase wird unter Rühren (20 U/Min.) in die Fryma zur heißen Fettphase gegeben. Kühlung wird gestartet mit laufendem Rührwerk (20 U/Min.). Homogenisation: 2 Min./2200 U. Vakuum wird abgelassen. Bei 35°C wird das Parfümöl einziehen gelassen. 1/2 Min. wird homogenisiert bei 700 U. Bis 27°C wird unter Rühren abgekühlt. Dann Homogenisation: 10 Min./4000 U. Rührerdrehzahl: 20 U/Min. Vakuum wird abgelassen. Bei 27°C wird die Lotion mittels Druckluft in einen sterilen Lagertank gepumpt (ca. 1600 U).

### Beispiel 2:

### Rezeptur 2: Hautschutzcreme (W/O Emulsion)

| | | | |
|---|---|---|---|
| Aqua (Wasser) | 42-46 | % | Lösungsmittel |
| Zinkoxid | 19-22 | % | Feststoff zum Abdecken und damit zum mechanischen Schutz der Haut (Hautschutzmittel) |
| Cethyl Ethylhexanoate | 6-10 | % | Weichmacher, Öl-Komponente |
| Decyl Cocoate | 5-9 | % | Weichmacher, Öl-Komponente |
| Paraffinum Liquidum | 4-8 | % | Öl-Komponente, Hautschutzmittel gegen wässrige Medien |
| Glycerin 85% | 1-4 | % | Anfeuchter/Feuchtig keitsspender |
| Cetyl PEG/PPG-10/1 Dimethicone | 0,5-4% | % | Emulgierungsmittel |
| Prunus Amygdalus Dulcis (Süßmandel) Öl | 0,5-4 | % | Weichmacher, Feuchtigkeitsspender/Pflegekomponente |
| Polyglyceryl-4 Isostearate | 0,5-4 | % | Emulgierungsmittel |
| Kaliumsorbat | | | |
| Natriumbenzoat | 0,5-2 | % | Konservierungsmittel |
| Zitronensäure | 0,5-4 | % | pH-Regulierer |
| Natriumhydroxid 50% | 0,2-08 | % | pH-Regulierer |
| Kreatin | 0,3-0,7 | % | Feuchtigkeitsspender |
| Natriumchlorid | 0,3-0,7 | % | Viskosität erhöhendes Mittel |
| Parfüm (Duftstoff) | 0,3-0,7 | % | Duftstoff |
| hydriertes Castor-Öl | 0,3-0,7 | % | Konsistenz-Faktor |
| Cera Alba (Bienenwachs) | 0,2-06 | % | Konsistenz-Faktor, Weichmacher |
| Leinölfettsäure | 0,2-0,6 | % | Feuchtigkeitsspender/ Öl-Komponente |
| Aminosäure-Komplex | 0,05-0,15 | % | Anfeuchter/Protein-Aufbaustoff |
| Rosmarinus Officinalis (Rosmarin) Blatt Extrakt | 0,005-0,015 | % | Antioxidationsmittel |
| Tocopherol | 0,001-0.004 | % | Vit. E/ Antioxidationsmittel/ Feuchtigkeitsspender |

Die Gewichtsanteile der einzelnen Komponenten werden so eingestellt, dass sich insgesamt 100 Gew.-% ergeben.

### Verfahrensanweisung

### a) Fettphase mit Zinkoxid

Tocopherol, Rosmarinextrakt, Leinölfettsäure werden destilliert, hydriertes Castor-Öl, Cera Alba, Polyglyceryl-4-Isostearat, Cetyl PEG/PPG-10/1 Dimethicon, Mandelöl, Paraffin dünnflüssig, Decyl Cocoat und Cethyl Ethylhexanoat werden in der Fryma vorgelegt und auf 85°C erwärmt. Bei einer Rührwerkeinstellung von 10 U/Min. werden alle Rohstoffe geschmolzen und die Temperatur etwa 15 Min. gehalten. Zinkoxid wird nach dem Homogenisator in die Fryma mit 1200 U/Min. eingezogen und homogenisiert: 3 Min./2000 U.

### b) Wasserphase

Der Unimix wird sterilisiert mit der minimal notwendigen Menge Wasser (ca. 150 kg); anschließend wird auf 55°C abgekühlt. Ein Teil heißes Wasser wird für die Vorlösungen herausgelassen: Natriumchlorid, Zitronensäure im heißen Wasser gelöst. Natronlauge 50 % wird im heißen Wasser gelöst. Aminosäure Komplex, Glycerol 85 % wird im heißen Wasser gelöst. Kaliumsorbat wird im heißen Wasser gelöst. Restliches Wasser, gereinigt - bis auf 10 % - wird in den Unimix eingezogen. Rührwerk 30 U/Min.. Die Vorlösungen werden in den Unimix eingezogen und gut gerührt, bis eine homogene Lösung entstanden ist (ca. 2 Min./1400 U).

Dann wird Kreatin eingezogen und die restlichen 10 % Wasser, gereinigt, nachgezogen. Homogenisation in 2 Min. bei 1400 U. Der pH Wert wird kontrolliert (Sollwert 4,8 - 5,1).

### c) Parfümölphase

Das Parfümöl wird abgewogen und bereitgestellt.

### d) Emulsionsbildung

Die kalte Wasserphase wird unter Rühren (20 U/Min.) in die Fryma zur heißen Fettphase gezogen. Kühlung wird gestartet mit laufendem Rührwerk (20 U/Min.). Homogenisation: 5 Min./2200 U. Vakuum wird abgelassen. Bei 35°C wird das Parfümöl einziehen gelassen. 1/2 Min. wird homogenisiert bei 1200 U. Bis 30°C wird unter Rühren abgekühlt. Dann Homogenisation: 6 Min./4100 U (bei 1.500 kg Ansatzgröße). Bis 29°C wird unter Rühren abgekühlt. Druckluft wird aufgebaut (1 bar). Bei 29°C wird die Creme mittels Druckluft (1 bar) und mit einer Homogenisatorleistung von 2000 bis 3000 U in einen sterilen Lagertank gepumpt. Hierbei wird das Vakuum abgelassen.

### Beispiel 3:

### Rezeptur 3: Transparente Hautschutzcreme (W/O/W Emulsion)

| | | | |
|---|---|---|---|
| Aqua (Wasser) | 60-80 | % | Lösungsmittel (vehiculum) |
| PARAFFINUM LIQUIDUM | 4-8 | % | Öl-Komponente, Hautschutzmittel gegen wässrige Medien |
| ISOPROPYL PALMITAT | 1-4 | % | Weichmacher |
| CETEARYL ALKOHOL | 1-4 | % | Viskosität erhöhendes Mittel |
| POLYGLYCERYL-2 DIPOLYHYDROXYSTEARAT | 1-4 | % | Haut-Konditionierung |
| PROPYLENGLYCOL | 1-4 | % | Lösungsvermittler, Anfeuchter |
| CETEARYL GLUCOSID | 1-4 | % | Emulgierung |
| C 12-15 ALKYL BENZOAT | 1-4 | % | Weichmacher, Haut-Konditionierer, Fett-Komponente |
| STEARINSÄURE | 0,5-3 | % | Matrixbilder, Emulgierungsmittel |
| PETROLATUM | 0,5-3 | % | Hautschutzmittel |
| BISABOLOL | 0,5-3 | % | Hautpflegemittel |
| PHENOXYETHANOL | 0,5-1,5 | % | Konservierungsmittel |
| PEG-30 DIPOLYHYDROXYSTEARAT | 0,1-0,8 | % | Emulgierungsmittel |
| PEG-40 STEARAT | 0,1-0,8 | % | Emulgierungsmittel |
| KREATIN | 0,05-0,15 | % | Feuchtigkeitsspender, |
| PRUNUS AMYGDALUS DULCIS Öl | 0,1-0,8 | % | Weichmacher, Feuchtigkeitsspender/Pflegekomponente |
| LEINÖLFETTSÄURE | 0,05-0,15 | % | Feuchtigkeitsspender//Öl-Komponente |
| PARFÜM (DUFTSTOFF) | 0,05-0,4 | % | Duftstoff |
| ROSMARINUS OFFICINALIS BLATT EXTRAKT | 0,01-0,05 | % | Antioxidationsmittel |
| METHYLPARABEN | 0,1-0,5 | % | Konservierungsmittel |
| PROPYLPARABEN | 0,01-0,04 | % | Konservierungsmittel |
| ETHYLPARABEN | 0,01-0,05 | % | Konservierungsmittel |
| ISOBUTYLPARABEN | 0,005-0,03 | % | Konservierungsmittel |
| BUTYLPARABEN | 0,01-0,05 | % | Konservierungsmittel |
| AMINOSÄURE-KOMPLEX | 0,05-0,15 | % | Anfeuchter/Protein-Aufbaustoff / |

Die Gewichtsanteile der einzelnen Komponenten werden so eingestellt, dass sich insgesamt 100 Gew.-% ergeben.

### Verfahrensanweisung

### a) Wasserphase

Demineralisiertes Wasser wird vorgelegt und Kreatin, Propylenglycol, PEG 40 Stearat und Aminosäuren darin dispergiert. Die Phase wird auf 80°C aufgeheizt, danach wird eine Mischung aus Phenoxyethanol, Methylparaben, Butylparaben, Ethylparaben, Propylparaben und Isobutylparaben zugegeben und homogen dispergiert.

### b) Fettphase

Nacheinander werden Cetearyl Glucoside, Polyglyceryl-2 Dipolyhydroxystearat, PEG-30 Dipolyhydroxystearat, Paraffinum Liquidum, Petrolatum, Isopropylpalmitat, C12-C15 Alkylbenzoat, Cetearylalkohol und Stearinsäure, Mandelöl, Leinölfettsäure und Rosmarinus Officinalis Blattextrakt vorgelegt, bei 80°C aufgeschmolzen und dabei homogen gemischt. Zuletzt wird bei 80°C Bisabolol zugegeben und homogen untergerührt.

### c) Parfümölphase

Das Parfümöl wird abgewogen und bereitgestellt.

### d) Emulsionsbildung

Die 80°C heiße Fettphase wird anschließend in die 80°C heiße Wasserphase überführt und dabei homogen untergerührt. Unter Vakuum wird nachgerührt und anschließend homogenisiert. Unter Rühren wird bis unter 30°C abgekühlt. Zum Schluss wird das Parfümöl untergerührt und das Produkt abgefüllt.

### Beispiel 4:

### Rezeptur 4: Hautprotektor (Schaum)

| | | | |
|---|---|---|---|
| Aqua (Wasser) | 60-80 | % | Lösungsmittel |
| Butan | 4-8 | % | Treibmittel |
| Harnstoff | 2-6 | % | Anfeuchter, Feuchtigkeitsspender |
| Stearinsäure | 2-6 | % | Matrixbildner, Emulgierungsmittel |
| Octyl Palmitat | 1-4 | % | Lösungsmittel/Trägermittel/Weich-macher/ Duftstoff-Fixierer |
| Propan | 1-4 | % | Treibmittel |
| Isobutan | 1-4 | % | Treibmittel |
| Palmitinsäure | 0,5-1,5 | % | Trübungsmittel/Emulgierungsmittel |
| Myristinsäure | 0,5-1,5 | % | Trübungsmittel/Tensid |
| Natriumlaurylsulfat/Natriummyristylsulfat/ Natriumcetylsulfate/Natrium-stearylsulfat/ Laureth 10 | 0,5-1,5 | % | Trübungsmittel/perlmutterartiges Pigment |
| Lecithin | 0,5-1,5 | % | Öl-Komponente, Öl-wiederherstellendes/rückfettendes Mittel (Weichmacher) |
| Mineralöl | 0,5-1,5 | % | |
| Alkohol | 0,5-1,5 | % | Hauterfrischungsmittel; Konservierungsmittel |
| Ethanolamin | 0,2-0,8 | % | Emulgierungsmittel/ pH-Regulierer |
| Panthenol | 0,2-0,8 | % | Anfeuchter (Feuchtigkeitsspender) |
| Prunus Dulcis Öl | 0,2-0,8 | % | Weichmacher, Feuchtigkeitsspender/Pfleqemittel |
| Leinölfettsäure | 0,2-0,8 | % | Feuchtigkeitsspender/Öl-Komponente |
| Kreatin | 0,2-0,8 | % | Feuchtigkeitsspender |
| Aminomethyl Propanol | 0,2-0,8 | % | pH-Regulierer |
| Propylenglycol | 0,38 | % | Lösungsvermittler, Anfeuchter |
| Sorbitan Trioleat | 0,05-0,15 | % | Emulgierungsmittel |
| Oleinsäure | 0,005-0,15 | % | Tensid/Feuchtigkeitsspender |
| Aminosäuren | 0,005-0,15 | % | Anfeuchter/Protein-Aufbaustoff |
| Glycerin | 0,005-0,15 | % | Anfeuchter, Feuchtigkeitsspender |
| Cocamide MEA | 0,01-0,09 | % | Schaum-stabilisierender und Konsistenz gebendes Mittel |

Die einzelnen Komponenten des Schaums werden so gewählt, dass sich insgesamt 100 Gew.-% ergeben.

### Verfahrensanweisung :

1. Cocamide MEA, Stearinsäure, Palmitinsäure, Oleinsäure und Myristinsäure werden bei ca. 60-70°C geschmolzen (Voransatz 1).
2. Demineralisiertes Wasser, Ammoniummethylpropanol, Ethanolamin, Glycerin und eine Mischung aus Natriumlaurylsulfat, Natriummyristylsulfat, Natriumcetylsulfat, Natriumstearylsulfate und Laureth 10 werden auf ca. 60-70°C erwärmt (Voransatz 2).
3. Die Schmelze aus Voransatz 1 wird über ein Sieb in das heiße Wasser (Voransatz 2) gegeben und gut gerührt.
4. Kühlen auf 40°C.
5. Anschließend werden die restlichen Rohstoffe Aminosäuren, Leinsamenöl und Mandelöl unter Rühren zugeben.
6. Zum Schluss werden Lecithin und Alkohol zugegeben.
7. Die Abfüllung erfolgt auf einer speziellen Aerosolabfülllinie. Die Aerosoldosen werden mit 86 g ± 1,7 g der Wirkstoffemulsion befüllt. Die Aerosoldosen werden mit dem Ventil bestückt und verkrimpt. Danach wird Propan/Butan mit 9,5 g ± 0,5 g durch das Ventil gefüllt. Anschließend werden die Dosen mit 7 bar Stickstoff beaufschlagt.

### Beispiel 5:

### Nachweis der Wirksamkeit

### 1. In vitro Rekonstruktion humaner Epidermis - Histologische Untersuchung

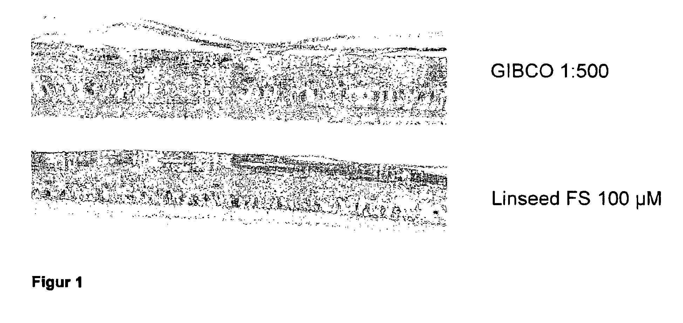

Die Überprüfung der Wirksamkeit von kosmetischen Produkten stößt auf verschiedene Schwierigkeiten. Aus ethischen Gründen können viele Versuche am Menschen nicht durchgeführt werden. Auch der Einsatz von Tieren zur Testung von Kosmetika stößt auf erhebliche Widerstände. Im Rahmen der vorliegenden Erfindung wurde daher ein in vitro-System verwendet, bei dem menschliche primäre Keratinozyten auf einer Polycarbonat-Filtermembran gezüchtet wurden, wobei die hierfür üblichen Wachstumsfaktoren und Zusatzstoffe zugesetzt wurden. Zunächst wurden die Zellen für etwa 4-5 Tage submers angezogen und dann in Kontakt mit der Luft-/Flüssig-Interphase gebracht. Die Zellkulturen wurden einmal mit und einmal ohne die Leinölfettsäuren in einem Standardnährmedium (GIBCO) angezogen. Dabei wurde im histologischen Schnitt ein deutlicher Unterschied festgestellt. In beiden Fällen wird eine Ausdifferenzierung der Basalzellen beobachtet, jedoch ist die gebildete Zellstruktur im Leinölfettsäure-haltigen Nährmedium kompakter. Figur 1 zeigt die Morphologie von in vitro rekonstruierter Epidermis nach 14 Tagen. Die obere Zellschicht wurde ohne Leinölfettsäuren angezogen, die untere in Gegenwart von 100 µM Leinölfettsäuren.

Die Übertragung dieses im in vitro Modell beobachteten Effekts auf die Anwendung an Altershaut führt dazu, dass sich eine kompakte Hautstruktur ausbildet, die von Vorteil ist, um eine Versorgung der äußeren Hautschichten mit Feuchtigkeit von innen zu ermöglichen. Die Verbesserung der Barrierewirkung des Wirkstoffkomplexes auf die Altershaut wird maßgeblich durch die Leinölfettsäuren verursacht.

### Beispiel 6:

Wie in Beispiel 5 beschrieben, wurden Keratinozyten angezogen. Die Zellen wurden einem Stress durch Zugabe eines Detergenz (1 %ige wässrige Lösung Triton X-100) ausgesetzt. Verglichen wurde auch hier die Lebensfähigkeit der Zellen, die einmal in Gegenwart von Leinölfettsäure (100 µM) angezogen wurden, mit Zellen, die in Nährmedien ohne Leinölfettsäure (GIBCO) angezogen wurden.

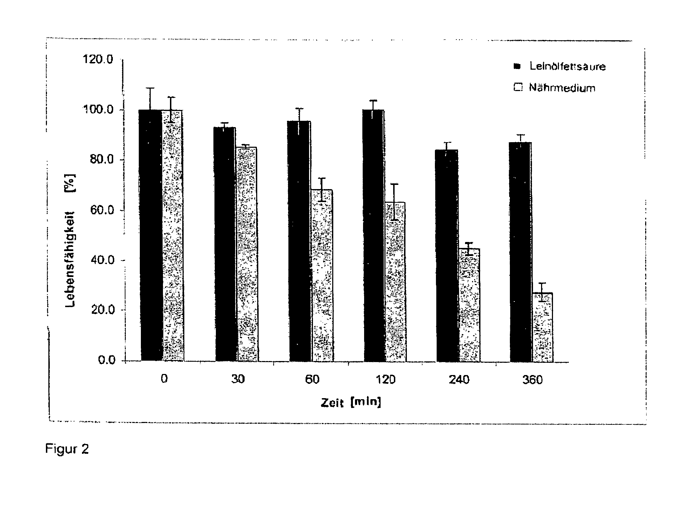

Werden die rekonstruierten Zellen einem Detergenz ausgesetzt, so wird ein signifikanter Verlust der Lebensfähigkeit der Zellen ( > 50 % ) nach 240 Minuten beobachtet, die im Standardnährmedium ausdifferenziert sind, wohingegen die Zellen aus einem Leinölfettsäure-haltigen Nährmedium wesentlich robuster gegenüber dem Detergenz sind und keinen signifikanten Rückgang der Lebensfähigkeit zeigen, sogar nicht nach 360 Minuten. Die Ergebnisse sind in Figur 2 dargestellt.

### Beispiel 7:

### Schutz der Haut vor wässrigen Schadstoffen

Die Rezepturen 1 bis 4 wurden an 16 hautgesunden Probanden getestet. Als Kontrolle diente eine 2,5 %ige Lösung von Natriumlaurylsulfat auf unbehandelter Haut ohne ein Hautschutzmittel.

### Testbeschreibung:

1. Tag bis 4. Tag
a) visuelle Beurteilung der Hautschädigung und Tewl-Messungen in den einzelnen Testfeldern
b) Auftragung der Testprodukte auf die Testfelder, 5 Minuten Einwirkzeit
c) okklusive Applikation des Schadstoffes in Finn-Kammern für 30 Minuten
d) Wiederholung der Schritte b) und c) nach 2-3 Stunden

5. Tag nur visuelle Beurteilung und TEWL-Messungen (TEWL = transepidermal water loss)

Eine Einteilung der Hautschädigung erfolgt in Rötung und Schuppung der Haut sowie der Bildung von Fissuren. Alle drei Merkmale werden im Verhältnis zur Kontrolle beurteilt. Die Einteilung erfolgt jeweils in 0,5er Schritten wobei der Wert "0" bedeutet, dass eine Hautschädigung nicht aufgetreten ist. Die Werte von Rötung, Schuppung und Fissuren werden addiert. Der Test wird für ein bestimmtes Testfeld abgebrochen, wenn aus ethischen Gesichtspunkten eine weitere Hautschädigung des Probanden nicht mehr zu verantworten ist (addierter Wert der Hautschädigung von ca. 2-3). Deshalb wird die Applikationsdauer eines Produktes mit in die Auswertung einbezogen.

### a) Ergebnisse visuelle Beurteilung:

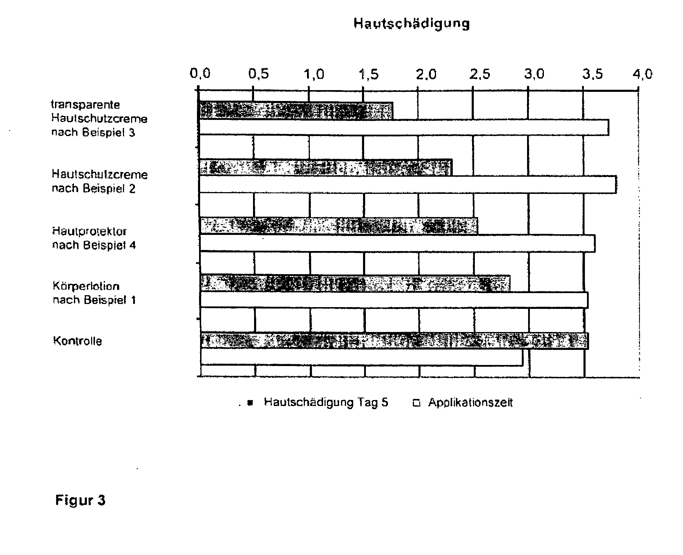

Alle Rezepturen 1 bis 4 haben vor der schädigenden Wirkung des Natriumlaurylsulfates geschützt. Die Hautschädigung war bei allen Testfeldern mit den Rezepturen 1 bis 4 niedriger als beim Kontrollwert. Dies korreliert mit einer längeren Applikationszeit der Produkte gegenüber Natriumlaurylsulfat. Den höchsten Schutz hat in diesem Test die transparente Hautschutzcreme (Rezeptur 3) bewirkt. Die Ergebnisse sind in Figur 3 dargestellt.

### b) Ergebnisse transepidermaler Wasserverlust (TEWL):

Die Messungen des Transepidermalen Wasserverlustes (TEWL) wurden mit Sonden der Firma Courage & Khazaka, Köln, durchgeführt. Bei den Testfeldern, bei denen der Test vorzeitig abgebrochen wurde, sind die Endwerte mit Hilfe einer linearen Regression bestimmt worden.

Der Standardschadstoff Natriumlaurylsulfat bewirkt eine Barriereschädigung der Haut, die zu einer erhöhten Verdunstung von Wasser durch die obersten Hautschichten führt. Im Durchschnitt steigt dieser Wert um 11 g/hm² an. Bei allen getesteten Rezepturen 1 bis 4 ist dieser Anstieg geringer. Der transepidermale Wasserverlust liegt bei den Rezepturen 1 bis 4 unter 10 g/hm², insbesondere zwischen 3 g/hm² und 9 g/hm². Die geringste Barriereschädigung der Haut wird durch die Vorbehandlung mit Hautschutzcreme (Rezeptur 2) bewirkt; der transepidermale Wasserverlust liegt bei etwa 4 g/hm².

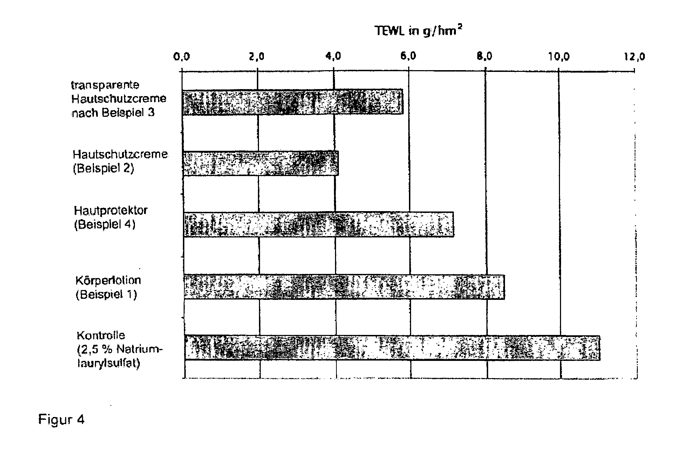

Die Ergebnisse des Versuchs sind in Figur 4 dargestellt.

Die Rezepturen 1 bis 4 zeigen alle eine Schutzwirkung gegenüber wässrigen Schadstoffen, getestet am Modellschadstoff Natriumlaurylsulfat. In der visuellen Beurteilung traten deutlich geringere Hautschädigungen als beim Kontrollwert auf. Die Barrierefunktion der Haut konnte durch die Verwendung des genannten Produkts gegenüber dem Standard besser aufrecht erhalten werden. Als besonders effektiv erwiesen sich die transparente Hautschutzcreme (Rezeptur 3) und die Hautschutzcreme (Rezeptur 2).

### Beispiel 8:

### Untersuchung der Wechselwirkung der transparenten Hautschutzcreme (Rezeptur 3) mit Inkontinenzprodukten

Bei der Anwendung topischer Schutz- und Pflegeprodukte bei Menschen, die auf Inkontinenzprodukte angewiesen sind, sollte eine Beeinträchtigung der Qualität dieser Inkontinenzprodukte vermieden werden. Es wurde daher am Beispiel der transparenten Hautschutzcreme untersucht, wie bzw. ob sie das Absorptionsverhalten von Inkontinenzprodukten beeinflusst.

Die eingecremte Haut kommt mit der im Tragezustand dem Körper zugewandten, obersten Lage (Topsheet) des Hygieneproduktes in Kontakt. Dabei besteht die Gefahr, dass von der eingecremten Haut Creme auf das Hygieneprodukt übertragen wird und die Durchlässigkeit dieser Lage negativ beeinflusst wird, und es dadurch letztendlich zu einem vorzeitigen Auslaufen des Produktes kommen kann.

Das Übertragen der Creme wird simuliert durch das direkte Aufbringen einer definierten Menge der erfindungsgemäßen Creme (Rezeptur 3) und von drei am Markt befindlichen Pflegeprodukten mit einer Lackierwalze aus Schaumstoff auf den Bereich des Verteilersystems. Als Testprodukte wurde das Inkontinenzprodukt "MoliCare Super Premium Air Active Size Medium" der Paul Hartmann AG verwendet.

### Probenvorbereitung:

Auf dem Inkontinenzartikel wird auf eine Fläche von 18cm mal 40cm (Bereich mit Verteilsystem: gelblicher Zellstoff, bis Vorderkante des Absobent Core = 720cm²) eine definierte Menge an Hautpflegemittel aufgetragen (2,5g = 34,72g/m²). Als Blindwert wird ein unbehandeltes Produkt verwendet.

Mit einer 11 cm Lackier-Schaumstoff-Walze wird eine definierte Menge Hautpflegemittel auf den vorbestimmten Kontaktbereich des Inkontinenz-Artikel verteilt und eingearbeitet (hierbei ist darauf zu achten, dass keine Creme-Nester entstehen). Das Aufbringen mit der Lackier-Schaumstoff-Walze ergibt eine sehr gleichmäßige Verteilung der Hautpflegemittel auch bei geringen Auftragsmengen.

### 1. Run-off Test:

Der Inkontinenzartikel wird auf eine schiefe Ebene gespannt. Am oberen Ende wird zweimal eine definierte Menge Testflüssigkeit über einen Zulauftrichter zugegeben. Am unteren Ende wird die abperlende Flüssigkeitsmenge aufgefangen und gewogen.

### 2. Einsickerzeiten (Acquisition time)/Rücknässung (Rewet) Test:

Der Inkontinenzartikel wird auf eine körperangepasste Testapparatur (KANGA Inko) angelegt, so dass der behandelte Bereich des Artikels mittig zum Zugabepunkt ist. (Seitliche Belastung: 4,5kg) Die Testapparatur wird seitlich mit der Unterseite auf eine Rampe mit einer Neigung von 35° gestellt (in Anlehnung an den Absorption Before Leakage (ABL-)Test der EDANA). Die untere Seitenkante hat hierbei auf der horizontalen Bodenfläche aufzuliegen. Über das Zugaberohr werden 100ml 0,9%iger NaCl-Lösung (gefärbt) gegeben und die Acquisitionszeit gemessen. Dies wird dreimal wiederholt (gesamte Flüssigkeitsmenge: 300ml), jeweils mit einer Wartezeit zwischen den Zugaben von 10 Minuten.

Nach jeder Wartezeit wird der Inkontinenzartikel aus dem KANGA genommen und flach auf eine Tischplatte gelegt. Ca. 40g Filterpapier (Macherey-Nagel MN617, Durchmesser 90 mm) werden mittig auf dem Zugabepunkt des Inkontinenzartikels aufgelegt und 5 Minuten mit einem Gewicht von 1.270 g belastet. Anschließend wird der Filterstapel zurückgewogen und so die Rücknässung ermittelt.

### a) Ergebnisse Run-off Test:

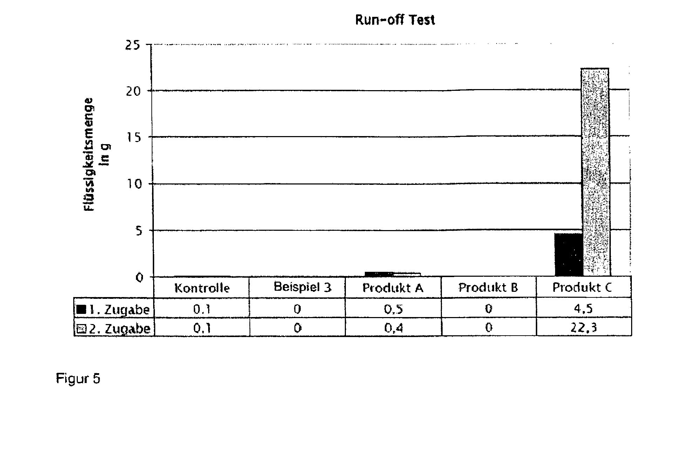

Die Kontrollmessung ergibt eine minimale Flüssigkeitsmenge, die von dem unbehandelten Top-Sheet abperlt. Die transparente Hautschutzcreme erhöht diese Menge nicht, beeinflusst die Qualität des Inkontinenzproduktes also nicht. Im Test waren auch drei Wettbewerbsprodukte, von denen nur eins (Produkt B) keinen Einfluss auf den Run-off Wert hatte. Produkt A führte zu einer leichten Erhöhung, Produkt C zu einer sehr starken Erhöhung der Flüssigkeitsmenge und damit zu einer erhöhten Gefahr des vorzeitigen Auslaufens des Hygieneproduktes. Die Ergebnisse sind in Figur 5 dargestellt.

### b) Ergebnisse Einsickerzeiten:

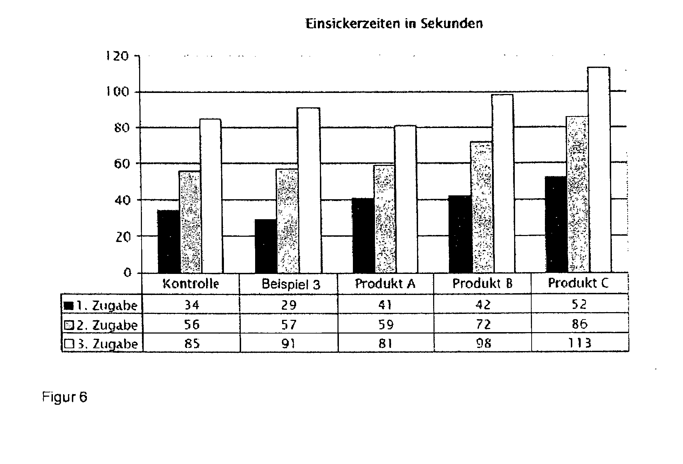

Gegenüber der Kontrollmessung zeigt ein mit transparenter Hautschutzcreme behandeltes Inkontinenzprodukt nur eine leicht längere 3. Einsickerzeit. Bei den mit Produkten B und C behandelten Windeln kommt es bei allen drei Zugaben zu deutlich längeren Zeiten, die die Flüssigkeit zum Eindringen benötigt. Die Ergebnisse sind in Figur 6 dargestellt.

### c) Ergebnisse Rücknässung:

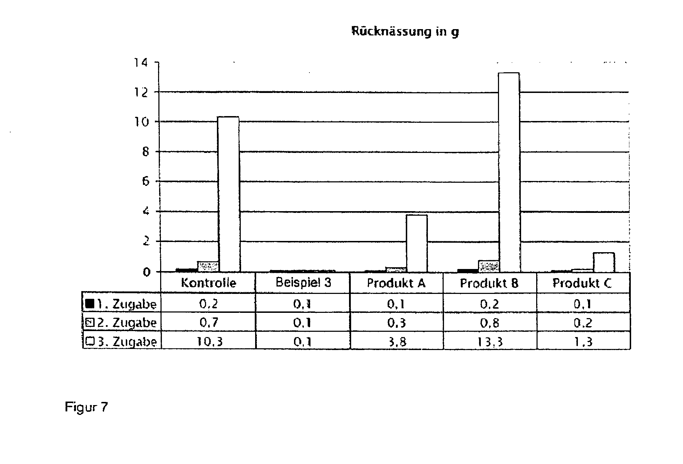

Bei den Kontrollmessungen steigt die von dem Inkontinenzprodukt nicht aufgenommene Flüssigkeitsmenge bei jeder Zugabe. Diese Tendenz wird auch bei allen Wettbewerbsprodukten A, B, C beobachtet, jedoch nicht bei der erfindungsgemäßen Zusammensetzung der transparenten Hautschutzcreme (Rezeptur 3). Die Produkte A und C verringern zwar auch die wieder abgegebene Menge an der Testflüssigkeit, aber nur die erfindungsgemäße Zusammensetzung nach Rezeptur 3 verhindert die Rücknässung fast vollständig. Dies wird in Figur 7 dargestellt.

### d) Zusammenfassung

Am Beispiel der transparenten Hautschutzcreme konnte gezeigt werden, dass die erfindungsgemäßen Zusammensetzungen keinen negativen Einfluss auf die Qualität der getesteten Inkontinenzprodukte haben, belegt an Run-off Tests sowie Messungen der Einsickerzeiten und der Rücknässung. Darüber hinaus konnte gezeigt werden, dass die Rücknässungseigenschaften der Inkontinenzprodukte positiv beeinflusst werden, derart, dass die Rücknässung fast vollständig unterbleibt. Dies wirkt sich wiederum positiv auf den Hautzustand der Hautpartien aus, die mit dem Inkontinenzartikel in Berührung kommen.

### Beispiel 9:

### Praxistest

In einem Praxistest wurden die Rezepturen 1, 3 und 4 von 90 examinierten Altenpflegern an 270 Bewohnern von Alten-/Pflege-/Behindertenheimen getestet, wobei jeder Altenpfleger drei Bewohner mit den Produkten behandelte, von denen mindestens zwei Bewohner an mindestens leichter Inkontinenz litten und bereits vor Beginn des Praxistests mehrmals pro Woche mit Körperpflegeprodukten gepflegt wurden. 64 % der teilnehmenden Heimbewohner litten an problematischen Hautzuständen.

Bei dem Praxistest wurden die zu untersuchenden Gruppen so gebildet, dass entweder der Schaum verwendet wurde oder die transparente Hautschutzcreme in Kombination mit der Körperlotion. Während des zweiwöchigen Testzeitraums wurden weitere, nicht erfindungsgemäße Produkte angewandt, insbesondere zur Reinigung (Waschlotion, Pflegebad, Shampoo, Reinigungsschaum, Feuchttücher), aber auch zum Schutz (Öl-Hautschutzspray) und zur Pflege der Haut (Hautfluidgel, Pflegeöl, Handcreme).

Sowohl die feuchtigkeitsspendende Wirkung, als auch die schützende Wirkung, die pflegende Wirkung und die Hautverträglichkeit der untersuchten Produkte wurde in mehr als 92 % aller Fälle mit gut bis sehr gut bewertet. Es wurde festgestellt, dass sich während der ersten Woche der Anwendung der Hautzustand bei 63 % der Heimbewohner deutlich verbesserte und nach zwei Wochen Anwendungsdauer bei 71 % der Heimbewohner der Hautzustand verbessert war. Dabei wurde abgefragt die Hautzustände: trocken, rissig/schuppend, juckend, gerötet, pergamentartig.

## Patentansprüche

1. Medizinische Hautschutz-Zusammensetzung, enthaltend eine Wirkstoffkombination für die Pflege der Altershaut, die aus folgenden Komponenten besteht:
a) 0,01 bis 20 Gew.-% Mandelöl,
b) 0,01 bis 1,5 Gew.-% Leinölfettsäuren,
c) 0,001 bis 0,5 Gew.-% Aminosäuren und
d) 0,01 bis 1,5 Gew.-% Kreatin, oder
die aus folgenden Komponenten besteht:
a) 0,1 bis 15 Gew.-% Mandelöl,
b) 0,05 bis 0,8 Gew.-% Fettsäuren pflanzlichen Ursprungs mit einem omega6 zu omega3 Fettsäure Verhältnis von 1 zu >1
c) 0,005 bis 0,15 Gew.-% Aminosäuren, ausgewählt aus einer oder mehrerer der Aminosäuren Arginin Arg, Glutamin Gln, Histidin His und Serin Ser und/oder mindestens einen Natural Moisturizing Factor, vorzugsweise Pyrrolidincarbonsäure PCA und/oder Urocaninsäure UCA
d) 0,05 bis 0,8 Gew.-% Kreatin,
wobei sich die Gewichtsprozent jeweils auf die Gesamtzusammensetzung beziehen,
**dadurch gekennzeichnet, dass** die medizinische Hautschutz-Zusammensetzung als Wasser-in-Öl-Emulsion (W/O) oder als Wasser-in-Öl-in-Wasser-Emulsion (W/O/W) vorliegt.

2. Medizinische Hautschutz-Zusammensetzung nach Anspruch 1, **dadurch gekennzeichnet, dass** das Mandelöl einen Gehalt von 70-80 % Ölsäure, 10-20 % Linolsäure sowie 5-10 % Palmitinsäure aufweist.

3. Medizinische Hautschutz-Zusammensetzung nach einem oder mehreren der vorherigen Ansprüche, wobei es sich bei den Leinölfettsäuren um ein Gemisch verschiedener gesättigter, ungesättigter sowie mehrfach ungesättigter Fettsäuren handelt, das vorzugsweise die folgenden Fettsäuren in den jeweils angegebenen relativen Anteilen enthält:
Palmitinsäure 4-8 %,
Stearinsäure 2-5 %,
Ölsäure 16-26 %,
Linolsäure 14-24 %,
Linolensäure 43-55 % sowie Fettsäuren mit Kohlenstoffketten, die mehr als 18
Kohlenstoffatome aufweisen 0,1-2 %.

4. Wirkstoffkombination nach einem oder mehreren der vorherigen Ansprüche, **dadurch gekennzeichnet, dass** die Aminosäuren (c) ausgewählt sind aus einer oder mehreren der Aminosäuren Arginin Arg, Glutamin Gln, Histidin His und Serin Ser und/oder mindestens einen Natural Moisturizing Factor, vorzugsweise Pyrrolidincarbonsäure PCA und/oder Urocaninsäure UCA.

5. Medizinische Hautschutz-Zusammensetzung nach Anspruch 1-4, **dadurch gekennzeichnet, dass** sie als Wasser-in-Öl-Emulsion, insbesondere in Form einer Körperlotion, vorliegt, wobei die Wasser-in-Öl-Emulsion zu einem Anteil von 1,0 bis 25 Gew.-%, vorzugsweise 2,5 bis 20 Gew.-%, weiter bevorzugt 5,0 bis 18 Gew.-% aus der Wirkstoffkombination besteht und, wobei sich das Verhältnis der Aminosäuren:Leinölfettsäuren:Kreatin:Mandelöl von 0,5:2:4:100 bis 0,5:4:5:100 bewegt.

6. Medizinische Hautschutz-Zusammensetzung nach Anspruch 1-4, **dadurch gekennzeichnet, dass** sie in einer Wasser-in-Öl-Emulsion, insbesondere in Form einer Hautschutzcreme vorliegt, die 0,5 bis 8,0 Gew.-%, vorzugsweise 0,75 bis 7,0 Gew.-%, weiter bevorzugt 1,0 bis 6,0 Gew.-% der Wirkstoffkombination, bezogen auf die Gesamtzusammensetzung, beinhaltet, wobei sich das Verhältnis der Komponenten der Wirkstoffkombination Aminosäure:Leinfettsäuren:Kreatin:Mandelöl von 0,5:4:6:10 bis 0,5:4:5:27 bewegt.

7. Medizinische Hautschutz-Zusammensetzung nach einem der Ansprüche 1-4, **dadurch gekennzeichnet, dass** sie in Form einer Wasser-in-Öl-in-Wasser-Emulsion, insbesondere in Form einer transparenten Hautschutzcreme vorliegt, die einen Anteil von 0,05 bis 2,0 Gew.-%, vorzugsweise 0,1 bis 1,7 Gew,%, weiter bevorzugt 0,2 bis 1,3 Gew.-% der Wirkstoffkombination, bezogen auf die Gesamtzusammensetzung, enthält und wobei sich das Verhältnis der Komponenten Aminosäuren:Leinölfettsäuren:Kreatin:Mandelöl von 0,5:1:1:2 bis 0,5:1:1:5 bewegt.

8. Medizinische Hautschutz-Zusammensetzung nach einem der Ansprüche 1-4, **dadurch gekennzeichnet, dass** sie in Form eines Schaumes mit 0,1 bis 4,0 Gew.-%, vorzugsweise 0,2 bis 3,5 Gew.-%, weiter bevorzugt 0,5 bis 3,0 Gew.-% der Wirkstoffkombination, bezogen auf die Gesamtzusammensetzung, vorliegt, wobei sich das Verhältnis der Komponenten Aminosäure:Leinölfettsäuren:Kreatin:Mandelöl im Bereich von 0,0125:1:1:1 bis 0,125:1:1:1 bewegt.

9. Medizinische Hautschutz-Zusammensetzung nach einem der Ansprüche 1 bis 8, zur topischen Anwendung auf Altershaut.

10. Medizinische Hautschutz-Zusammensetzung zur Verwendung nach Anspruch 9 auf durch Bettlägerigkeit und/oder Harn- und/oder Stuhlinkontinenz und/oder Dekubitus und andere Wunderkrankungen beanspruchter Altershaut.

11. Medizinische Hautschutz-Zusammensetzung zur Verwendung nach einem der Ansprüche 9 oder 10 zur Körperregion-spezifisch topischen Anwendung auf Altershaut, insbesondere zur Applikation am ganzen Körper und/oder auf die periläsionale Haut und/oder den Intimbereich, insbesondere den durch Inkontinenz beanspruchten Intimbereich.

12. Medizinische Hautschutz-Zusammensetzung zur Verwendung nach einem der Ansprüche 9 bis 11 in Kombination mit der Verwendung von absorbierenden Hygieneprodukten, insbesondere Inkontinenzartikeln, Wundauflagen und Krankenunterlagen.

13. Medizinische Hautschutz-Zusammensetzung nach einem oder mehreren der Ansprüche 1 bis 8 zur topischen Anwendung auf durch Bettlägerigkeit und/oder Harn- und/oder Stuhlinkontinenz und/oder Dekubitus und anderer Wunderkrankungen beanspruchter Altershaut.

14. Medizinische Hautschutz-Zusammensetzung nach einem oder mehreren der Ansprüche 1 bis 8 zur Körperregion-spezifisch topischen Anwendung auf Altershaut, insbesondere zur Applikation am ganzen Körper und/oder auf die periläsionale Haut und/oder den Intimbereich, insbesondere den durch Inkontinenz beanspruchten Intimbereich, in Kombination mit der Verwendung von absorbierenden Hygieneprodukten, insbesondere Inkontinenzartikeln, Wundauflagen und Krankenunterlagen.

15. Absorbierender Hygieneartikel, **dadurch gekennzeichnet, dass** er eine medizinische Hautschutz-Zusammensetzung nach einem der Ansprüche 1 bis 8 in Form einer Beschichtung auf der der Haut des Benutzers zugewandten Seite des Hygieneartikels aufweist.

## Claims

1. A medicinal skin protection composition containing an active ingredient combination for the care of aging skin consisting of the following components:
a) 0.01 to 20% by weight of almond oil,
b) 0.01 to 1.5% by weight of linseed oil fatty acids,
c) 0.001 to 0.5% by weight of amino acids, and
d) 0.01 to 1.5% by weight of creatine, or
consisting of the following components:
a) 0.1 to 15% by weight of almond oil,
b) 0.05 to 0.8% by weight of fatty acids of vegetable origin with an omega6 to omega3 fatty acid ratio of 1 to >1,
c) 0.005 to 0.15% by weight of amino acids selected from one or more of the amino acids arginine Arg, glutamine Gln, histidine His, and serine Ser and/or at least one natural moisturizing factor, preferably pyrrolidine carboxylic acid PCA and/or urocanic acid UCA,
d) 0.05 to 0.8% by weight of creatine,
wherein the weight percentages each relate to the total composition,
**characterized in that** the medicinal skin protection composition is present as water-in-oil emulsion (W/O) or as water-in-oil-in-water emulsion (W/O/W).

2. The medicinal skin protection composition according to claim 1, **characterized in that** the almond oil has a content of 70-80% oleic acid, 10-20 % linoleic acid as well as 5-10% palmitic acid.

3. The medicinal skin protection composition according to one or more of the preceding claims, wherein the linseed oil fatty acids are a mixture of various saturated, unsaturated as well as polyunsaturated fatty acids, preferably containing the following fatty acids in the respective given relative proportions:
palmitic acid 4-8%,
stearic acid 2-5%,
oleic acid 16-26%,
linoleic acid 14-24%,
linolenic acid 43-55% as well as fatty acids with carbon chains having more than 18 carbon atoms 0.1-2%.

4. An active ingredient combination according to one or more of the preceding claims, **characterized in that** the amino acids (c) are selected from one or more of the amino acids arginine Arg, glutamine Gln, histidine His, and serine Ser and/or at least one natural moisturizing factor, preferably pyrrolidine carboxylic acid PCA and/or urocanic acid UCA.

5. The medicinal skin protection composition according to claim 1-4, **characterized in that** it is present as water-in-oil emulsion, in particular in the form of a body lotion, wherein the water-in-oil emulsion in a proportion of 1.0 to 25% by weight, preferably 2.5 to 20% by weight, further preferred 5.0 to 18% by weight consists of the active ingredient combination and wherein the ratio of amino acids:linseed oil fatty acids:creatine:almond oil is from 0.5:2:4:100 to 0.5:4:5:100.

6. The medicinal skin protection composition according to claim 1-4, **characterized in that** it is present as a water-in-oil emulsion, in particular in the form of a skin protection cream containing 0.5 to 8.0% by weight, preferably 0.75 to 7.0% by weight, further preferred 1.0 to 6.0% by weight of the active ingredient combination, based on the total composition, wherein the ratio of the components of the active ingredient combination amino acids:linseed fatty acid:creatine:almond oil is from 0.5:4:6:10 to 0.5:4:5:27.

7. The medicinal skin protection composition according to anyone of claims 1-4, **characterized in that** it is present in the form of a water-in-oil-in-water emulsion, in particular in the form of a transparent skin protection cream containing a proportion of from 0.05 to 2.0% by weight, preferably 0.1 to 1.7% by weight, further preferred 0.2 to 1.3% by weight of the active ingredient combination, based on the total composition, and wherein the ratio of the components amino acids:linseed fatty acids:creatine:almond oil is from 0.5:1:1:2 to 0.5:1:1:5.

8. The medicinal skin protection composition according to anyone of claims 1-4, **characterized in that** it is present in the form of a foam having 0.1 to 4.0% by weight, preferably 0.2 to 3.5% by weight, further preferred 0.5 to 3.0% by weight of the active ingredient combination, based on the total composition, wherein the ratio of the components amino acid:linseed oil fatty acids:creatine:almond oil is in the range of from 0.0125:1:1:1 to 0.125:1:1:1.

9. The medicinal skin protection composition according to anyone of claims 1 to 8 for the topical application on aging skin.

10. The medicinal skin protection composition for use according to claim 9 on aging skin strained by bed confinement and/or urinary and/or fecal incontinence and/or decubitus and other wound diseases.

11. The medicinal skin protection composition for use according to anyone of claims 9 or 10 for the body region-specific topical application on aging skin, in particular for application to the whole body and/or to the perilesional skin and/or the genitals, in particular the genitals strained by incontinence.

12. The medicinal skin protection composition for use according to anyone of claims 9 to 11 in combination with the use of absorbing healthcare products, in particular incontinence articles, primary wound dressings, and patient under pads.

13. The medicinal skin protection composition according to one or more of claims 1 to 8 for the topical application on aging skin strained by bed confinement and/or urinary and/or fecal incontinence and/or decubitus and other wound diseases.

14. The medicinal skin protection composition according to one or more of claims 1 to 8 for the body region-specific topical application on aging skin, in particular for the application to the whole body and/or to the perilesional skin and/or the genitals, in particular the genitals strained by incontinence, in combination with the use of absorbing healthcare products, in particular incontinence articles, primary wound dressings, and patient under pads.

15. An absorbing healthcare article **characterized in that** it has a medicinal skin protection composition according to anyone of claims 1 to 8 in the form of a coating on the side of the healthcare article that faces the skin of the user.

## Revendications

1. Composition médicale de protection de la peau, contenant une combinaison de principes actifs destinée au soin des peaux matures, laquelle est constituée des composants qui suivent :
a) 0,01 à 20 % en poids d'huile d'amande ;
b) 0,01 à 1,5 % en poids d'acides gras d'huile de lin ;
c) 0,001 à 0,5 % en poids d'acides aminés ; et
d) 0,01 à 1,5 % en poids de créatine ; ou
laquelle est constituée des composants qui suivent :
a) 0,1 à 15 % en poids d'huile d'amande ;
b) 0,05 à 0,8 % en poids d'acides gras d'origine végétale présentant un rapport entre acide gras oméga-6 et acide gras oméga-3 de 1 à > 1 ;
c) 0,005 à 0,15 % en poids d'acides aminés, choisis parmi l'un ou plusieurs des acides aminés que sont l'arginine Arg, la glutamine Gln, l'histidine His et la sérine Ser et/ou parmi au moins un facteur naturel d'hydratation (Natural Moisturizing Factor), de préférence l'acide carboxylique de pyrrolidine PCA et/ou l'acide urocanique UCA ;
d) 0,05 à 0,8 % en poids de créatine,
où les pourcentages en poids se rapportent respectivement à la composition globale,
**caractérisée en ce que** la composition médicale de protection de la peau se présente sous la forme d'une émulsion eau-dans-huile (E/H) ou sous la forme d'une émulsion eau-dans-huile-dans-eau (E/H/E).

2. Composition médicale de protection de la peau selon la revendication 1, **caractérisée en ce que** l'huile d'amande présente une teneur de 70-80 % d'acide oléique, 10-20 % d'acide linoléique ainsi que 5-10 % d'acide palmitique.

3. Composition médicale de protection de la peau selon l'une quelconque ou plusieurs des revendications précédentes, où les acides gras d'huile de lin sont un mélange de différents acides gras saturés, insaturés ainsi que polyinsaturés, qui contient de préférence les acides gras qui suivent dans les proportions relatives respectivement indiquées :
de l'acide palmitique 4-8 %,
de l'acide stéarique 2-5 %,
de l'acide oléique 16-26 %,
de l'acide linoléique 14-24 %,
de l'acide linolénique 43-55 % ainsi que des acides gras comprenant des chaînes carbonées, qui présentent plus de 18 atomes de carbone 0,1-2 %.

4. Combinaison de principes actifs selon l'une quelconque ou plusieurs des revendications précédentes, **caractérisée en ce que** les acides aminés c) sont choisis parmi l'un ou plusieurs des acides aminés que sont l'arginine Arg, la glutamine Gln, l'histidine His et la sérine Ser et/ou au moins parmi un facteur naturel hydratant, de préférence l'acide carboxylique de pyrrolidine PCA et/ou l'acide urocanique UCA.

5. Composition médicale de protection de la peau selon la revendication 1-4, **caractérisée en ce qu'**elle se présente sous la forme d'une émulsion eau-dans-huile, en particulier sous la forme d'une lotion corporelle, où l'émulsion eau-dans-huile est constituée d'une proportion allant de 1,0 à 25 % en poids, de préférence de 2,5 à 20 % en poids, de manière davantage préférée de 5,0 à 18 % en poids de la combinaison de principes actifs, où le rapport acides aminés:acides gras d'huile de lin:créatine:huile d'amande varie de 0,5:2:4:100 à 0,5:4:5:100.

6. Composition médicale de protection de la peau selon la revendication 1-4, **caractérisée en ce qu'**elle se présente sous la forme d'une émulsion eau-dans-huile, en particulier sous la forme d'une crème de protection de la peau, qui contient 0,5 à 8,0 % en poids, de préférence 0,75 à 7,0 % en poids, de manière davantage préférée 1,0 à 6,0 % en poids de la combinaison de principes actifs, par rapport à la composition globale, où le rapport des composants de la combinaison de principes actifs acide aminé:acides gras d'huile de lin:créatine:huile d'amande varie de 0,5:4:6:10 à 0,5:4:5:27.

7. Composition médicale de protection de la peau selon l'une quelconque des revendications 1-4, **caractérisée en ce qu'**elle se présente sous la forme d'une émulsion eau-dans-huile-dans-eau, en particulier sous la forme d'une crème de protection de la peau transparente, qui contient une proportion allant de 0,05 à 2,0 % en poids, de préférence de 0,1 à 1,7 % en poids, de manière davantage préférée de 0,2 à 1,3 % en poids de la combinaison de principes actifs, par rapport à la composition globale, et où le rapport des composants acides aminés:acides gras d'huile de lin:créatine:huile d'amande varie de 0,5:1:1:2 à 0,5:1:1:5.

8. Composition médicale de protection de la peau selon l'une quelconque des revendications 1-4, **caractérisée en ce qu'**elle se présente sous la forme d'une mousse comprenant 0,1 à 4,0 % en poids, de préférence 0,2 à 3,5 % en poids, de manière davantage préférée 0,5 à 3,0 % en poids de la combinaison de principes actifs, par rapport à la composition globale, où le rapport des composants acide aminé:acides gras d'huile de lin:créatine:huile d'amande varie dans la plage allant de 0,0125:1:1:1 à 0,125:1:1:1.

9. Composition médicale de protection de la peau selon l'une quelconque des revendications 1 à 8, destinée à être appliquée de manière topique sur les peaux matures.

10. Composition médicale de protection de la peau destinée à être utilisée selon la revendication 9 sur les peaux matures affectées par l'alitement et/ou par l'incontinence urinaire et/ou fécale et/ou par le décubitus et autres complications liées aux plaies.

11. Composition médicale de protection de la peau destinée à être utilisée selon l'une quelconque des revendications 9 ou 10 destinée à être appliquée de manière topique et spécifique à une zone corporelle sur les peaux matures, en particulier destinée à être appliquée sur tout le corps et/ou sur la peau périlésionnelle et/ou sur les parties intimes, en particulier les parties intimes affectées par l'incontinence.

12. Composition médicale de protection de la peau destinée à être utilisée selon l'une quelconque des revendications 9 à 11 en combinaison avec l'utilisation de produits d'hygiène absorbants, en particulier des articles pour l'incontinence, des pansements et des alèses.

13. Composition médicale de protection de la peau selon l'une quelconque ou plusieurs des revendications 1 à 8 destinée à être appliquée de manière topique sur les peaux matures affectées par l'alitement et/ou l'incontinence urinaire et/ou fécale et/ou par le décubitus et d'autres complications liées aux plaies.

14. Composition médicale de protection de la peau selon l'une quelconque ou plusieurs des revendications 1 à 8, destinée à être appliquée de manière topique et spécifique à une zone corporelle sur les peaux matures, en particulier destinée à être appliquée sur tout le corps et/ou sur la peau périlésionnelle et/ou sur les parties intimes, en particulier les parties intimes affectées par l'incontinence, en combinaison avec l'utilisation de produits d'hygiène absorbants, en particulier des articles pour l'incontinence, des pansements et des alèses.

15. Article d'hygiène absorbant, **caractérisé en ce qu'**il présente une composition médicale de protection de la peau selon l'une quelconque des revendications 1 à 8, se présentant sous la forme d'un revêtement sur le côté, tourné vers la peau de l'utilisateur, de l'article d'hygiène.
